# EUROPEAN PATENT SPECIFICATION

(11) **EP 1 446 418 B1**
(45) Date of publication and mention of the grant of the patent: **23.05.2012**
(21) Application number: 02773859.0
(22) Date of filing: 22.10.2002
(51) Int. Cl.: C07K 5/08, C07K 5/09, A61K 38/06, A61K 47/48

(54) **INTEGRIN TARGETING COMPOUNDS**
AUF INTEGRIN ZIELENDE VERBINDUNGEN
COMPOSES DE CIBLAGE DE L'INTEGRINE

(30) Priority: 22.10.2001 US 343799 P; 19.09.2002 US 412519 P
(43) Date of publication of application: 18.08.2004
(73) Proprietor: THE SCRIPPS RESEARCH INSTITUTE, La Jolla, CA 92037 (US)
(72) Inventor: BARBAS, Carlos, F., Solana Beach, CA 92075 (US); RADER, Christoph, San Diego, CA 92122 (US); SINHA, Subhash, C., San Diego, CA 92130 (US)
(74) Representative: Fisher, Adrian John
(86) International application number: PCT/US2002/033866
(87) International publication number: WO 2003/034995

(56) References cited:
- WO-A-03/059251
- WO-A1-97/26250
- US-B1- 6 265 540
- SHABAT D ET AL: "In vivo activity in a catalytic antibody-prodrug system: Antibody catalyzed etoposide prodrug activation for selective chemotherapy." PROCEEDINGS OF THE NATIONAL ACADEMY OF SCIENCES OF THE UNITED STATES OF AMERICA. 19 JUN 2001, vol. 98, no. 13, 19 June 2001 (2001-06-19), pages 7528-7533, XP002372640 ISSN: 0027-8424
- SCHALLY A V ET AL: "Cancer chemotherapy based on targeting of cytotoxic peptide conjugates to their receptors on tumors." EUROPEAN JOURNAL OF ENDOCRINOLOGY / EUROPEAN FEDERATION OF ENDOCRINE SOCIETIES. JUL 1999, vol. 141, no. 1, July 1999 (1999-07), pages 1-14, XP002372641 ISSN: 0804-4643
- LIST B ET AL: "Aldol sensors for the rapid generation of tunable fluorescence by antibody catalysis" PROCEEDINGS OF THE NATIONAL ACADEMY OF SCIENCES OF THE UNITED STATES OF AMERICA 1998 UNITED STATES, vol. 95, no. 26, 1998, pages 15351-15355, XP002372642 ISSN: 0027-8424
- HALLAHAN D.E. ET AL.: 'Targeting drug delivery to radiation-induced neoantigens in tumor microvasculature' JOURNAL OF CONTROLLED RELEASE vol. 74, July 2001, pages 183 - 191, XP004297523
- POPKOV M. ET AL.: "Small molecule drug avtivity in melanoma models may be dramatically enhanced with an antibody effector", INT. J. CANCER, vol. 119, 1 January 2006 (2006-01-01), pages 1194-1207,

## Description

### BACKGROUND OF THE INVENTION

The invention relates to integrin targeting compounds and methods of making and using the compounds. The use of potent chemotherapeutics has some distinct limitations mainly related to the toxicity of the drug. The dose of drug that is required for therapy often renders patients susceptible to potentially fatal infections, cardiac toxicity and other side effects. Improved understanding of the biology of cancer has led to more specific "targeted therapies." Antigens expressed on the surface of diseased cells (e.g., tumor cells) form the basis of "monoclonal antibody directed drug delivery" approaches. Unfortunately, however, tumor antigens are often down regulated due to drug resistance: this limits the effectiveness of the "monoclonal antibody directed drug delivery" approaches. On the other hand, target molecules expressed on the surface of tumor endothelial cells are readily accessible to targeting molecules circulating in the blood. Furthermore, in contrast to tumor cells, tumor endothelial cells are genetically stable and are unlikely to be down regulated due to drug resistance. Hence, molecules expressed on the surface of tumor endothelial cells appear to be an appropriate receptor for "directed drug delivery."

The present invention solves problems of the art by providing novel compounds for targeting integrin expressing cells. Such compounds have diagnostic and therapeutic applications in cancer and other diseases.

### BRIEF SUMMARY OF THE INVENTION

The invention relates to an integrin targeting compound, comprising at least one integrin targeting component covalently linked to a linear or branched linker which is covalently linked to at least one functional component, wherein said integrin targeting component is an RGD peptidomimetic, and wherein the at least one functional component is an aldolase antibody and the at least one integrin targeting component is covalently linked via the linker to the combining site of the antibody.

In some embodiments, the integrin targeting component is specific for an integrin such as αᵥβ₃ or αᵥβ₅. The core structures of preferred RGD peptidomimetics are disclosed for use in particular targeting compound embodiments.

The integrin targeting compounds of the invention confer various benefits over the components themselves. For example, the functional component may generally extend the half-life of a smaller sized targeting component in vivo. Also, the biological potency or other biological feature of an integrin targeting component may be modified by the addition of effector function(s) provided by a functional component such as an antibody. In addition, the integrin targeting component, through its increased size conferred by linkage to the functional component, may enable the targeting agent to function in new capacities.

Also provided are methods of producing the integrin targeting compounds of the invention. In one embodiment, an integrin targeting component-linker compound is prepared which includes an integrin targeting component and a linker that includes a reactive group for covalent reaction with a susceptible reactive moiety of the functional component. In another approach, a functional component-linker compound is prepared that includes a functional component and a linker that includes a reactive group for covalent reaction with a susceptible reactive moiety of an integrin targeting component. In yet another approach, the targeting component and the functional component are linked to one of a linker with a reactive groups or linker with a susceptible moiety so that the targeting compound forms when the two linkers covalently bond.

Further disclosed are integrin targeting component-linker compounds and functional component-linker compounds for covalently linking an integrin targeting component to a functional component. In some embodiments, the linker includes a reactive group for covalently linking to the other of the components. In some embodiments, the linker reactive group is a ketone, a diketone, a beta lactam, a succinimide active ester, haloketone, a lactone, an anhydride, an epoxide, an aldehyde or a maleimide.

Various chemical features of the integrin targeting component-linker compounds and functional component linker compounds are described. In one embodiment, the linker has the general formula X - Y - Z wherein X is a linear or branched connecting chain of atoms comprising any of C, H, N, O, P, S, Si, F, Cl, Br, and I, or a salt thereof, and comprising a repeating ether unit of between 2-100 units; Y is optional and is a single or fused 5 or 6 membered homo- or heterocarbocylic saturated or unsaturated ring located within 1-20 atoms of Z; and Z is a reactive group for covalently linking the one or more targeting agents to susceptible moiety such as a side chain of a reactive amino acid. The targeting component or functional component may be linked to X or Y or to X and Y when multiple components are included in the targeting compound.

Additionally disclosed are methods of delivering a functional component to integrin associated cells, tissue of an individual. The method includes administering to the individual an integrin targeting compound of the invention. In some embodiments of the method, the functional component is a therapeutic agent which includes the functional component.

Still further provided is an integrin targeting compound of the invention for use in the treatment of a disease or condition that involves integrin in an individual. A therapeutically effective amount of an integrin targeting compound of the invention that includes a functional component that is a therapeutic agent is administered to the individual. The disease or condition is susceptible to the therapeutic agent such that a reduction or prevention of the symptoms associated with the disease or condition is effected. In some embodiments; the disease or condition involves a defect in angiogenesis, bone metabolism, inflammation or cell growth.

The invention further provides pharmaceutical compositions or medicaments that include an integrin targeting compound of the invention and a pharmaceutically acceptable carrier.

### BRIEF DESCRIPTION OF THE DRAWINGS

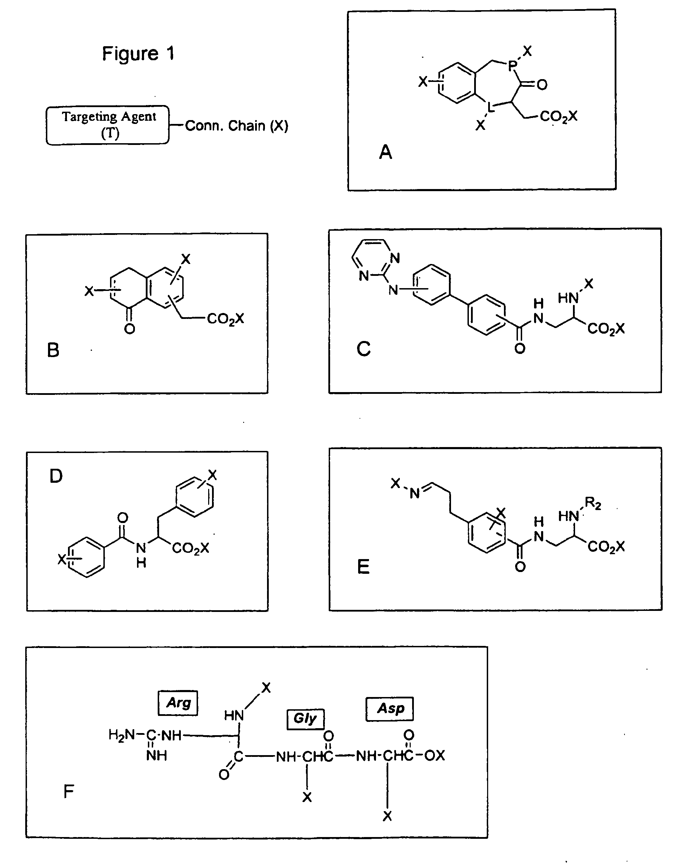

FIG. 1 shows exemplary integrin targeting agents of which Panels A-E are RGD peptidomimetic while Panel F is an RGD peptide. The core structures are from the following: U.S. Patent No. 6,335,330 (Panel A), U.S. Patent No. 5,693,636 (Panel B), U.S. Patent No. 6,040,311 (Panel C), and U.S. Patent No. 6,001,117 (Panel E).

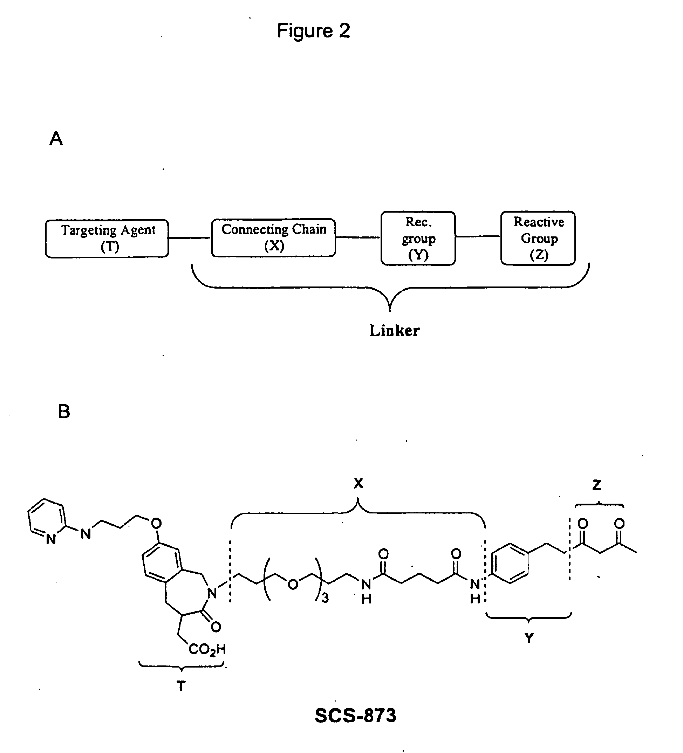

FIG. 2 is a general linker design (Panel A) and specific embodiment (Panel B; SCS-873) shown in association with a targeting agent.

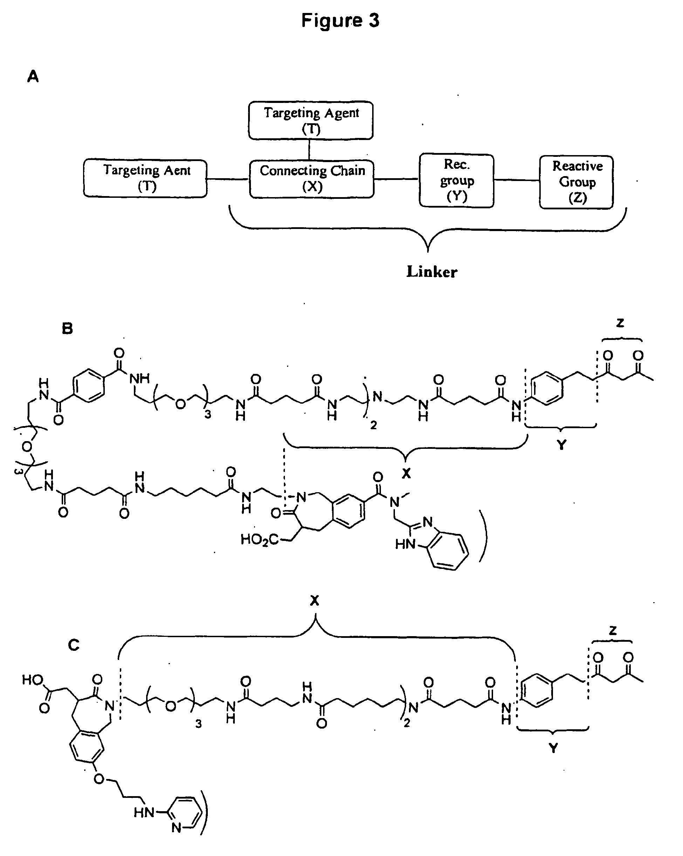

FIG. 3 shows a general scheme of an embodiment of a targeting agent-linker compound with a branched linker and two identical targeting agents (Panel A) with specific embodiments in Panel B (integrin targeting agent diketo linker; compound 26), and Panel C (integrin targeting agent diketo linker; compound 27). The branch point is in the connecting chain portion of the linker.

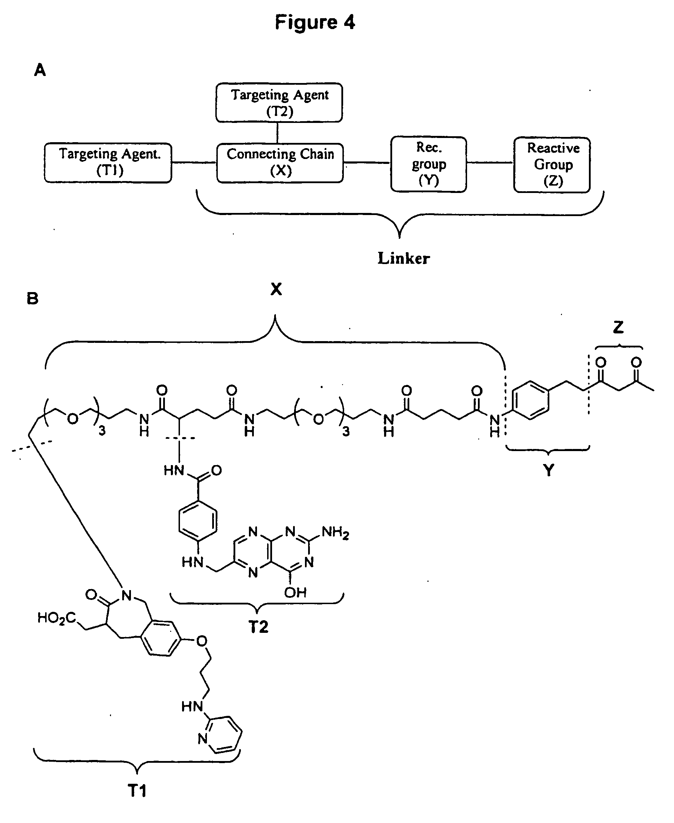

FIG. 4 shows a general scheme of an embodiment of a targeting agent-linker compound with a branched linker and two different targeting agents (Panel A) with a specific embodiment in Panel B (integrin targeting and folate targeting agent diketo linker; compound 28). The branch point is in the connecting chain portion of the linker. T1 is an integrin targeting component while T2 is a folate receptor targeting component.

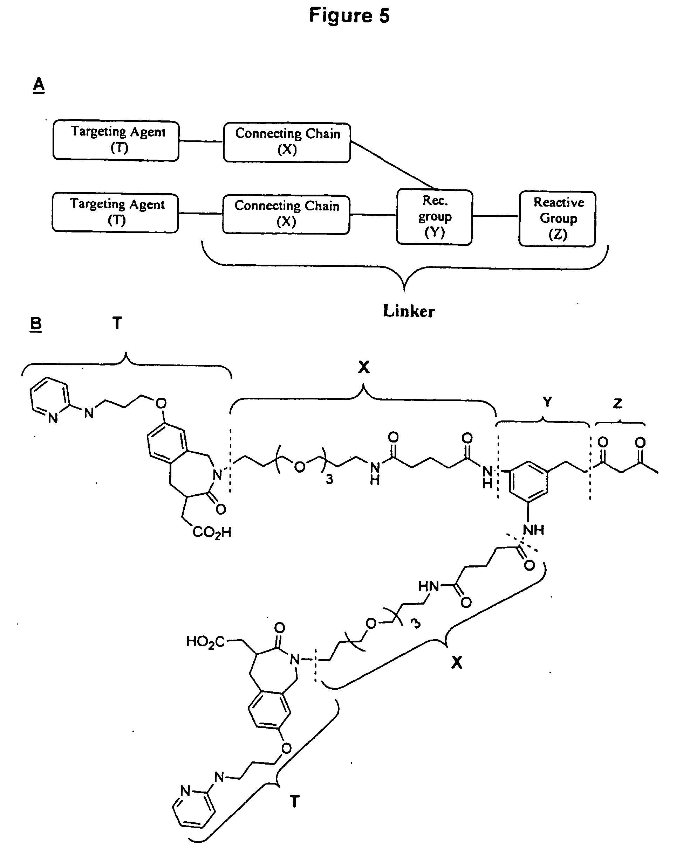

FIG. 5 shows a general scheme of an embodiment of a targeting agent-linker compound with a branched linker and two different targeting agents (Panel A) with a specific embodiment in Panel B (integrin targeting agent diketo linker; compound 29). The branch point is in the recognition group portion of the linker.

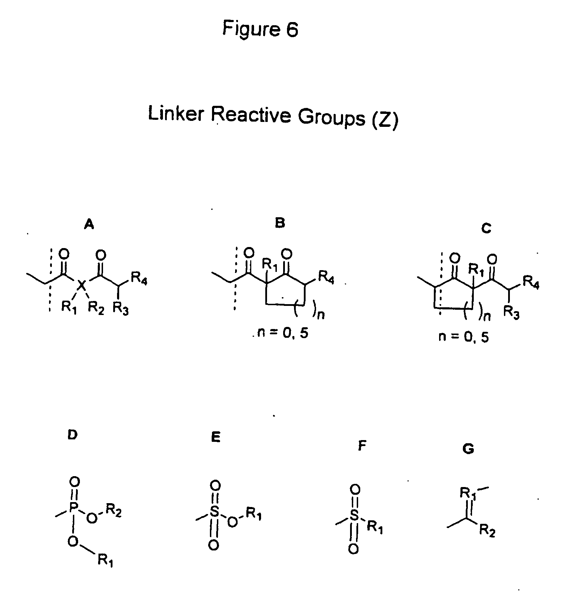

FIG. 6 shows the structure of linker reactive groups. Structures A-C form reversible covalent bonds with reactive nucleophilic group (e.g. lysine or cysteine side chain) in the combining site of an antibody (structure A could form an irreversible covalent bond X is N and if R₁ and R₃ form part of a cyclic structure). R₁ and R₂ and R₃ in structures A-C represent substituents which can be C, H, N, O, P, S, Si, halogen (F, Cl, Br, I) or a salt thereof. X is N, C, Si, or any other heteroatom. These substituents may also include a group such as an alkyl, alkenyl, alkynyl, oxoalkyl, oxoalkenyl, oxoalkynyl, aminoalkyl, aminoalkenyl, aminoalkynyl, sulfoalkyl, sulfoalkenyl, or sulfoalkynyl group, phosphoalkyl, phosphoalkenyl, phosphoalkynyl group. R₂ and R₃ could be cyclic as exemplified in structures B and C while X could be a heteroatom. Structures D-G form nonreversible covalent bonds with reactive nucleophilic group (e.g. lysine or cysteine side chain) in the combining site of an antibody. In these structures, R₁ and R₂ represent C, O N, halide and leaving groups such as mesyl or tosyl.

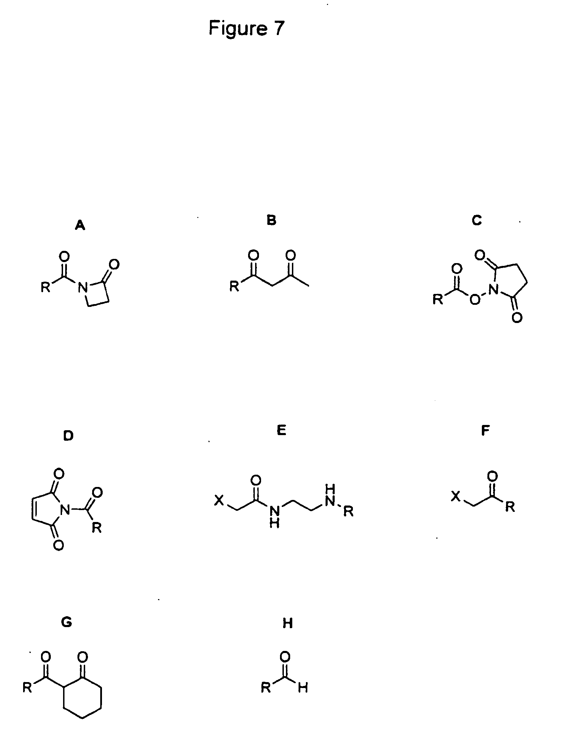

FIG. 7 shows various electrophiles suitable for reactive modification with a reactive amino acid side chain of an antibody. Key: (A) acyl beta-lactam; (B) simple diketone; (C) succinimide active ester; (D) maleimide; (E) haloacetamide with linker; (F) haloketone; (G) cyclohexyl diketone; and (H) aldehyde. R refers to other structure that may include a targeting agent, linker or antibody, while X refers to halogen.

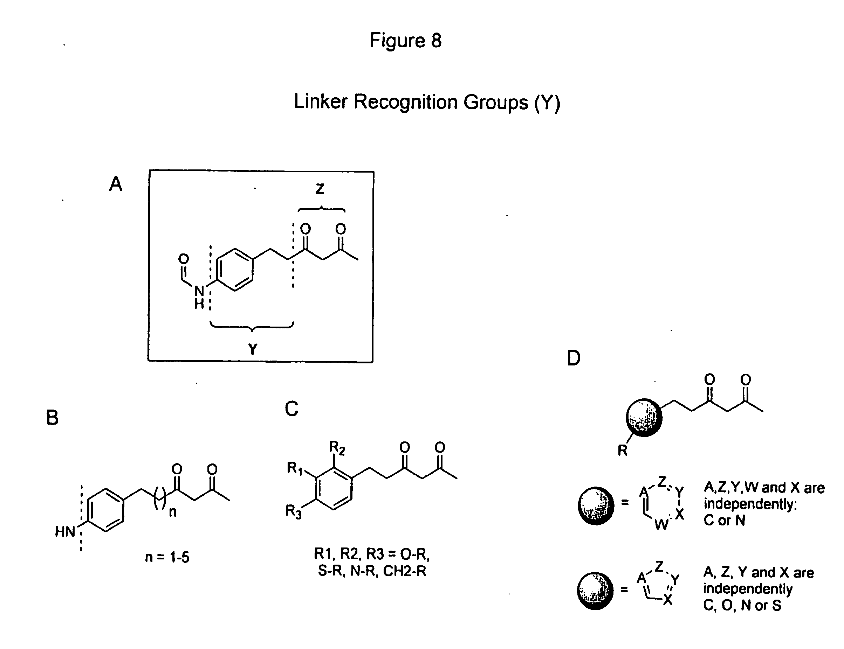

FIG. 8 shows the structure of linker recognition group (Y), situated between the reactive group portion and the connecting chain portion of the linker. Panel A shows the relationship of the recognition group Y within the linker (see FIG. 2). Panels B-D show distance of Y from Z, substituents on the ring and ring member atoms.

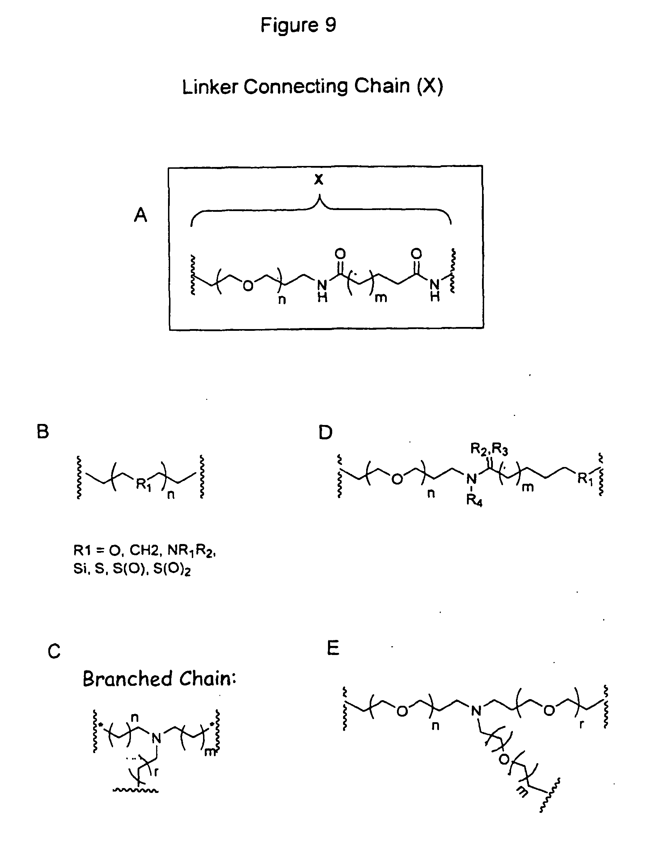

FIG. 9 shows the structure of the linker connecting chain (X), which directly attaches at one end to the targeting agent as shown in Panel A (see FIG. 2). Substituents R₂ to R₄ can be C, H, N, O, P, S, Si, halogen (F, Cl, Br, I) or a salt thereof, and may include a group such as an alkyl, alkenyl, alkynyl, oxoalkyl, oxoalkenyl, oxoalkynyl, aminoalkyl, aminoalkenyl, aminoalkynyl, sulfoalkyl, sulfoalkenyl, sulfoalkynyl group, phosphoalkyl, phosphoalkenyl, phosphoalkynyl as well as a carbocyclic or heterocyclic mono or fused saturated or unsaturated ring structure. In the connecting chain in structures B and C, n, r or m is 1-100. In structures D and E, n is 1, 2, 4, or more preferably is 3.

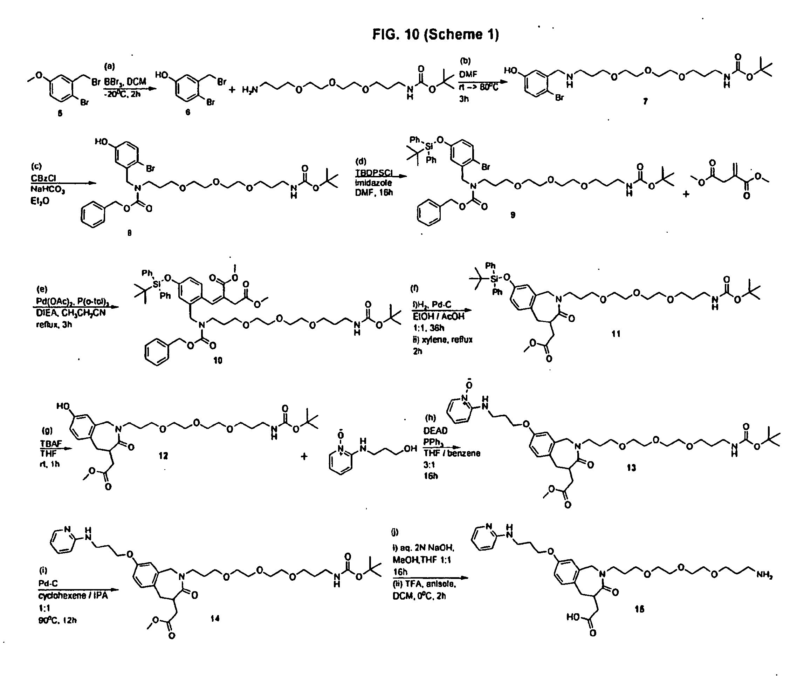

FIG. 10 shows Scheme 1, a synthetic scheme for the amine precursor of SCS-873, targeting agent 3 or SCS-amine. Key: (a) BBr₃, CH₂Cl₂, -20 °C, 2h; (b) DMF, rt to 80°C, 3h; (c) BnCOCl, sat. aq. NaHCO₃, ether; (d) TBDPSiCl, imidazole, DMF, 16h; (e) Pd(OAc)₂, (o-tol)₃P, *i*-Pr₂EtN, CH₃CH₂CN, reflux, 3h; (f) 20 % (w/w) Pd-C (10%), H₂, EtOH-AcOH (1:1), 36h; (g) TBAF, THF, rt, 1h; (h) DEAD, PPh₃, THF-benzene (3:1), 16h; (i) 20 % (w/w) Pd-C (10%), cyclohexene-*i*-PrOH (1:1), 90°C, 12h; (j) i. aq. 2N NaOH, MeOH-THF (1:1), 16h, ii. TFAA, anisole, CH₂Cl₂, 0°C, 2h.

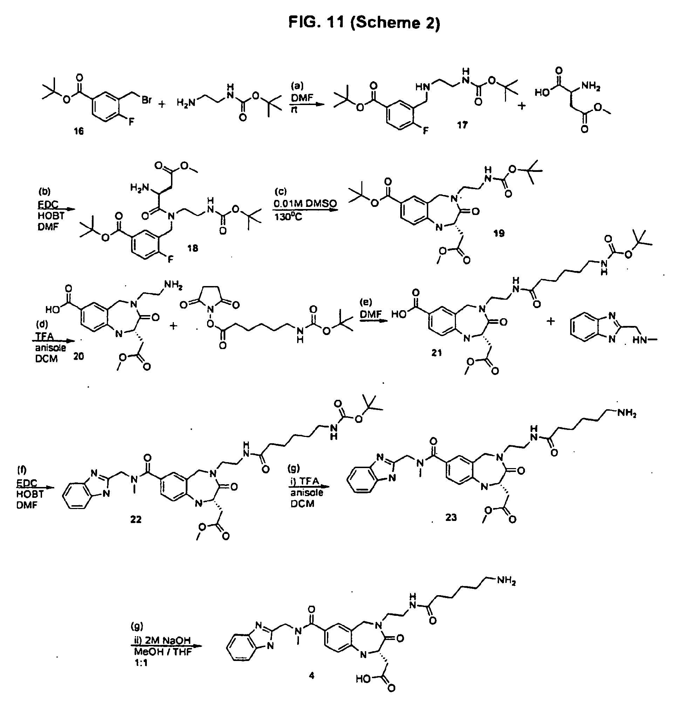

FIG. 11 shows Scheme 2, a synthetic scheme for making Compound 4, (R = Butoxycarboxyaminohexanoyl-derivative). Key: (a) DMF, rt; (b) EDC, HOBT, DMF; (c) 0.01 M in DMSO, 130 °C; (d) TFAA, anisole, dichloromethane; (e) DMF; (f) EDC, HOBT, DMF; (g) (i) step d, (ii) 2M NaOH, MeOH-THF (1:1).

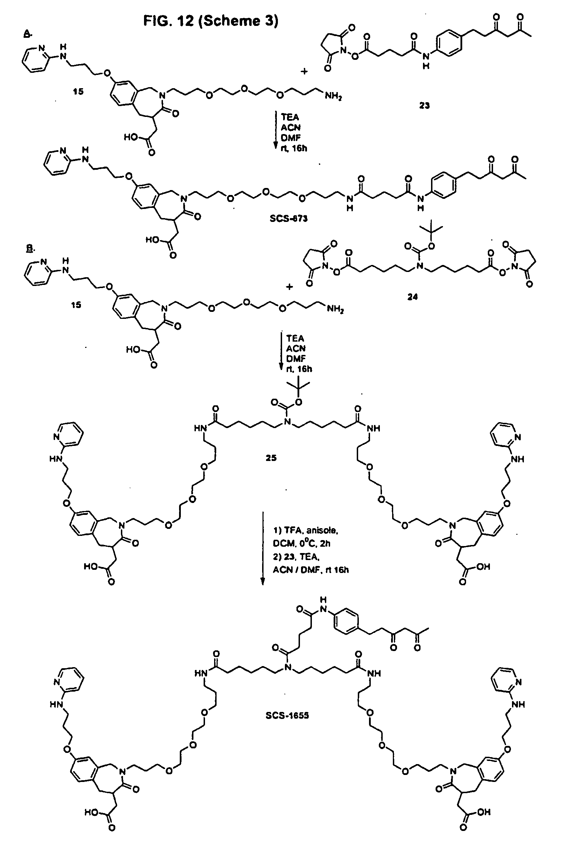

FIG. 12 shows Scheme 3, a synthetic scheme for making compounds SCS-873 and SCS-1655.

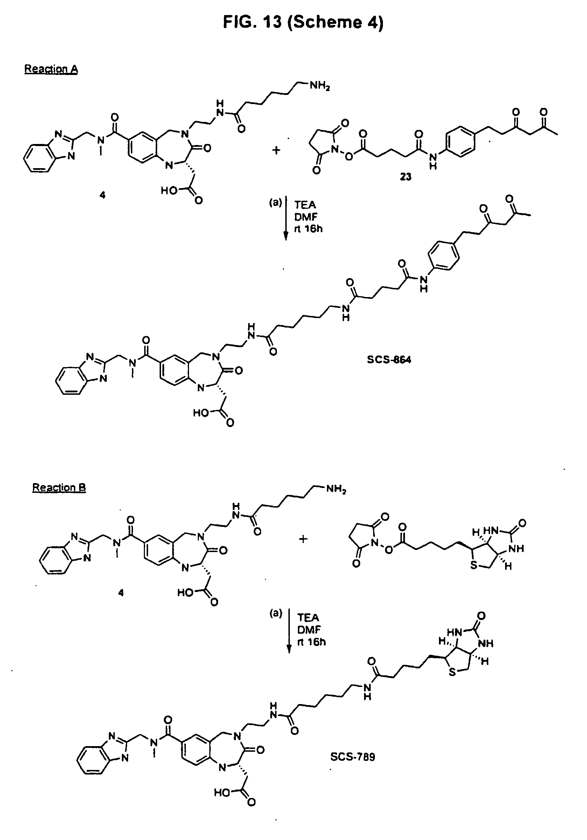

FIG. 13 shows Scheme 4, a synthetic scheme for making Compounds SCS-864 and SCS-789. Key: (a) Et3N, DMF, rt, 16h.

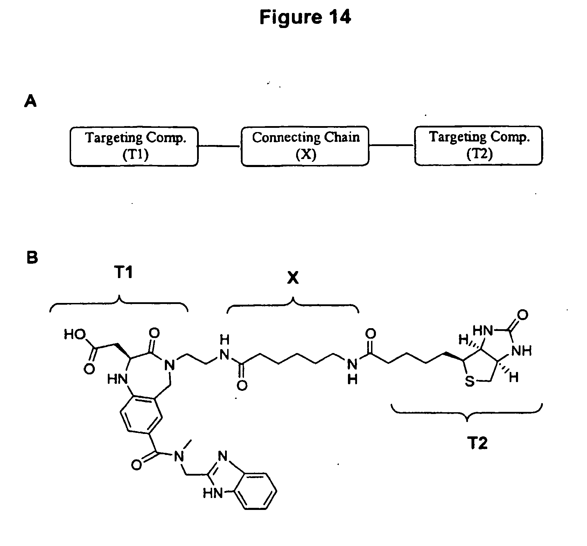

FIG. 14 shows an embodiment whereby two targeting components are linked to a single linker. T1 is an integrin targeting component and T2 is biotin (compound 30).

### DETAILED DESCRIPTION OF THE INVENTION

The present invention provides integrin targeting compounds useful for presentation to a particular integrin target such as a cell or tissue. The targeting component operates to situate the compound at the target site. Integrin targeting compounds comprise at least two components: a targeting component and a functional component which is an adolase antibody. The components are operatively linked such that each component retains its activity. The targeting component is an RGD peptidomimetic. Typically, the targeting component specifically binds to the integrin target typically through a ligand/receptor relationship. Such ligand/receptor relationships are well known in the art. Preferably the integrin target is on the surface of the target cell or tissue. The integrin target may also be associated with a particular condition such a pathological condition.

A targeting component targets to one or more integrins. The targeting component can be either an agonist or antagonist to the integrin or bind without any biological activity. In one embodiment, the integrin is αᵥβ₃ or αᵥβ₅. Integrins are heterodimeric transmembrane glycoprotein complexes that function in cellular adhesion events and signal transduction processes. Integrin αᵥβ₃ is expressed on numerous cells and has been shown to mediate several biologically relevant processes, including adhesion of osteoclasts to the bone matrix, migration of vascular smooth muscle cells, and angiogenesis. Integrin αᵥβ₃ antagonists may be employed in the treatment of several human diseases, including diseases involving neovascularization, such as rheumatoid arthritis, cancer, ocular diseases.

The integrin targeting component of integrin targeting compounds of the disclosure may be a small molecule organic compound of about 5,000 daltons or less such as a drug or pharmaceutical, which is an integrin antagonist or agonist. An integrin agonist or antagonist also can be a protein, peptide, peptidomimetic, glycoprotein, proteoglycan, lipid, phospholipid, lipopolysaccharide, glycolipid, nucleic acid, proteoglycan, carbohydrate, and the like. The terms "polypeptide", "peptide," and "protein" are used interchangeably to refer to a polymer of amino acid residues. The terms apply to amino acid polymers in which one or more amino acid residue is a synthetic chemical analogue (e.g., para-methyl-tyrosine, para-chloro-phenylanine, and the like) of a corresponding naturally occurring amino acid, as well as to naturally occurring amino acid polymers. Amino acids can be in the L or D form so long as the binding function of the peptide is maintained. Peptides can be of variable length, generally between about 4 and 200 amino acids. Peptides may be cyclic, having an intramolecular bond between two non-adjacent amino acids within the peptide, e.g., backbone to backbone, side-chain to backbone and side-chain to side-chain cyclization. Cyclic peptides can be prepared by methods well know in the art. See e.g., U.S. Pat. No. 6,013,625.
WO 9014103 teaches an RGD-antibody which is operatively attached to an integrin-binding polypeptide of sequence CGGAGAGRGDSP
US 171588 discloses murine monoclonal antibodies directed against integrins
Hallahan D.E. et Al., Journal of Controlled Release 74, (2001), 183-191, discloses peptides within fibrinogen that bind to α_{2b} β₃ labeled with ¹³¹I.
Shabat D. et Al., PNAS (2001), 98, 7528-7533, discloses the use of the catalytic aldolase antibody 38C2 for in situ activation of an anticancer etoposide prodrug.

As used herein, reference to "Arg-Gly-Asp peptide" or "RGD peptide" is intended to refer to a peptide having one or more Arg-Gly-Asp containing sequences which may function as a binding site for a receptor of the "Arg-Gly-Asp family of receptors", e.g., an integrin. Integrins, which comprise and alpha and a beta subunit, include numerous types including α₁β₁,α₂β₁, α₃β₁, α₄β₁, α₅β₁, α₆β₁. α₇β₁, α₈β₁, α₉β₁, α₁β₁, α₆β₄, α₄β₇, α_{D}β₂, α_{D}β₂, α_{L}β₂, α_{M}β₂,αᵥβ₁,αᵥβ₃, αᵥβ₅, αᵥβ₆, αᵥβ₈, αₓβ₂, α_{IIb}β₃, α_{IELb}β₇, and the like. The sequence RGD is present in several matrix proteins and is the target for cell binding to matrix by integrins. Platelets contain a large amount of RGD-cell surface receptors of the protein GP II_{b}/IIIₐ, which is primarily responsible, through interaction with other platelets and with the endothelial surface of injured blood vessels, for the development of coronary artery thrombosis. The term RGD peptide also includes peptides with amino acids that are functional equivalents (e.g., RLD or KGD) of RGD peptide provided that they interact with the same RGD receptor. Peptides containing RGD sequences can be synthesized from amino acids by means well known in the art, using, for example, an automated peptide synthesizer, such as those manufactured by Applied Biosystems, Inc., Foster City, California.

The integrin targeting component is a peptidomimetic agonist or antagonist, which preferably is a peptidomimetic agonist or antagonist of an RGD peptide As used herein the term "peptidomimetic" is a compound containing non-peptidic structural elements that are capable of mimicking or antagonizing the biological action(s) of a natural parent peptide. A peptidomimetic of an RGD peptide is an organic molecule that retains similar peptide chain pharmacophore groups of the RGD amino acid sequence but lacks amino acids or peptide bonds in the binding site sequence. A "pharmacophore" is a particular three-dimensional arrangement of functional groups that are required for a compound to produce a particular response or have a desired activity. The term "RGD peptidomimetic" is intended to refer to a compound that comprises a molecule containing the RGD pharmacophores supported by an organic/non-peptide structure. It will be understood that an RGD peptidomimetic may be part of a larger molecule that itself includes conventional or modified amino acids linked by peptide bonds.

RGD peptidomimetics are well known in the art, and have been described with respect to integrins such as GPIIb/IIIa, αᵥβ3 and αᵥβ₅ (See, e.g., Miller et al., J. Med. Chem. 2000, 43:22-26; and International Patent Publications WO 0110867, WO 9915178, WO 9915170, WO 9815278, WO 9814192, WO 0035887, WO 9906049, WO 9724119 and WO 9600730; see also Kumar et al., Cancer Res. 61:2232-2238 (2000)). Many such compounds are specific for more than one integrin. RGD peptidomimetics are generally based on a core or template (also referred to as "fibrinogen receptor antagonist template"), to which are linked by way of spacers to an acidic group at one end and a basic group at the other end of the core. The acidic group is generally a carboxylic acid functionality while the basic group is generally a N-containing moiety such as an amidine or guanidine. Typically, the core structure adds a form of rigid spacing between the acidic moiety and the basic nitrogen moiety, and contains one or more ring structures (e.g., pyridine, indazole, etc.) or amide bonds for this purpose. For a fibrinogen receptor antagonist, generally, about twelve to fifteen, more preferably thirteen or fourteen, intervening covalent bonds are present (via the shortest intramolecular path) between the acidic group of the RGD peptidomimetic and a nitrogen of the basic group. The number of intervening covalent bonds between the acidic and basic moiety is generally shorter, two to five, preferably three or four, for a vitronectin receptor antagonist. The particular core may be chosen to obtain the proper spacing between the acidic moiety of the fibrinogen antagonist template and the nitrogen atom of the pyridine. Generally, a fibrinogen antagonist will have an intramolecular distance of about 16 angstroms (1.6 nm) between the acidic moiety (e.g., the atom which gives up the proton or accepts the electron pair) and the basic moiety (e.g., which accepts a proton or donates an electron pair), while a vitronectin antagonist will have about 14 angstroms (1.4 nm) between the respective acidic and basic centers. Further description for converting from a fibrinogen receptor mimetic to a vitronectin receptor mimetic can be found in U.S. Pat. No. 6,159,964.

The peptidomimetic RGD core can comprise a 5-11 membered aromatic or nonaromatic mono- or polycyclic ring system containing 0 to 6 double bonds, and containing 0 to 6 heteroatoms chosen from N, O and S. The ring system may be unsubstituted or may be substituted on a carbon or nitrogen atom. Preferred core structures with suitable substituents useful for vitronectin binding include monocyclic and bicyclic groups, such as benzazapine described in WO 98/14192, benzdiazapine described in U.S. 6,239,168, and fused tricyclics described in U.S. 6,008,213.

U.S. Pat. No. 6,159,964 contains an extensive list of references in Table 1 of that document which disclose RGD peptidomimetic cores structures (referred to as fibrinogen templates) which can be used for prepraring RGD peptidomimetics. Preferred vitronectin RGD and fibronectin RGD peptidomimetics are disclosed in U.S. Patent Nos. 6,335,330; 5,977,101; 6,088,213; 6,069,158; 6,191,304; 6,239,138; 6,159,964; 6,117,910; 6,117,866; 6,008,214; 6,127,359; 5,939,412; 5,693,636; 6,403,578; 6,387,895; 6,268,378; 6,218,387; 6,207,663; 6,011,045; 5,990,145; 6,399,620; 6,322,770; 6,017,925; 5,981,546; 5,952,341; 6,413,955; 6,340,679; 6313,119; 6,268,378; 6,211,184; 6,066,648; 5,843,906; 6,251,944; 5,952,381; 5,852,210; 5,811,441; 6,114,328; 5,849,736; 5,446,056; 5,756,441; 6,028,087; 6,037,343; 5,795,893; 5,726,192; 5,741,804; 5,470,849; 6,319,937; 6,172,256; 5,773,644; 6,028,223; 6,232, 308; 6,322,770; 5,760,028.

Exemplary RGD peptidomimetic integrin targeting agents are shown below as compounds 1, 2, and 3 can be used for preparing an intregrin targeting compound of the present invention. In the three compounds, the linker is attached as indicated to the nitrogen of the seven membered ring. Other RGD peptidomimetic integrin targeting agents include compound 31, wherein P and L or carbon or nitrogen. The linker may be R1 or R2 while the R3 group includes a basic group such as an -NH group. In some embodiments, the R3 group is as shown in structures 1, 2, or 3. In some embodiments, the R3 group includes a heterocyclic group such a benzimidazole, imidazole, pyridine group. In some such embodiments, the R3 group is a alkoxy group, such as a propoxy group, that is substituted with a heterocarbyl group that is substituted with an alkylamine group, such as a methylamino group or the like, whereas in other embodiments, the R3 group is an alkoxy group, such as a propoxy group substituted with a heterocyclylamino group, such as with a pyridinylamino group or the like such as a 2-pyridinylamino group. In other embodiments R3 is a group of formula -C(=O)Rb where Rb is selected from -N(alkyl)-alkyl-heterocyclyl groups such as -N(Me)-CH2-benzimidazole groups.

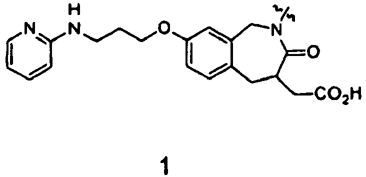

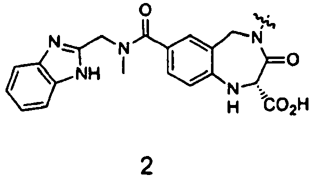

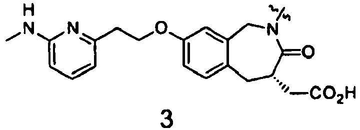

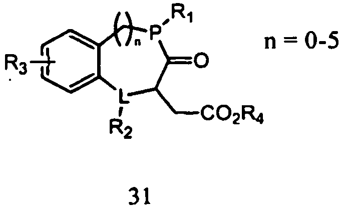

Other exemplary integrin peptidomimetic targeting agents and a peptide targeting agent are shown in FIG. 1. The linker may be any of R₁, R₂, R₃, while R₄ may be a linker or a hydrolyzable group such as alkyl, alkenyl, alkynyl, oxoalkyl, oxoalkenyl, oxoalkynyl, aminoalkyl, aminoalkenyl, aminoalkynyl, sulfoalkyl, sulfoalkenyl, or sulfoalkynyl group, phosphoalkyl, phosphoalkenyl, phosphoalkynyl group. One of skill in the art art will readily appreciate that other integrin agonist and antagonist mimetics can additionally be used in targeting compounds of the present invention.

A targeting compound of the invention can contain more than one targeting component. In such an embodiment, one of the targeting components targets an integrin as disclosed above. The second targeting component can target another entity. Exemplary such entities are chemokine receptors such as CCR5, interleukin or cytokine receptors, vitamin receptors such as the folate receptor, cancer markers such as prostate specific antigen, carcinoembryonic antigen, HER-2, viral markers such as HIV gp41, or a peptide hormone receptor. Linear or branched linkers may be used for preparing dual agent targeting compounds. Exemplary branched linker designs are shown in FIGs. 3-5.

An exemplary such dual targeting compound (Compound 32) is shown below, where the compound targets both integrin and the folate receptor. Another dual targeting compound that uses a single linear linker and targets integrin and avidin is shown in FIG. 14 (compound 30). Compound 30, which has a shorter linker than in other examples, has several uses. For example, compound 30 can be used in conjunction with a detectably labeled avidin or streptavidin to test cells for expression of the integrin target reactive with T1. In another embodiment, Compound 30 can be administered in vivo together with avidin or streptavidin labeled with a suitable therapeutic or imaging agent.

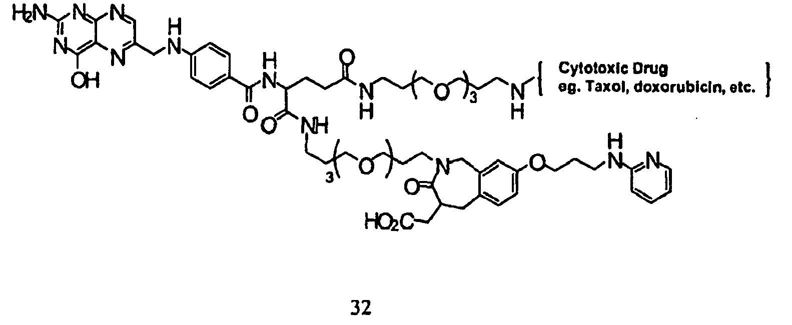

A targeting compound of the invention can contain more than one functional component. In such an embodiment, one of the functional component is an aldolase antibody. The other functional components may have one or more biological activities, each activity characterized as a detectable biological affect on the functioning of a cell organ or organism A targeting compound may, however, be a pure binding compound without biological activity.

The other functional components of a targeting compound can be any structure having a desired biological activity toward a cell or tissue associated with integrin. In some embodiments, the functional component is not a metal chelating structure with or without the associated metal ion, e.g., a radiometal ion. In some embodiments, the functional component is not a lipid. The functional component may include any of a number of biologically active structures well known in the art. Biological agents suitable as the functional component of the targeting compounds include, small molecule drugs (a pharmaceutical organic compound of about 5,000 daltons or less), organic molecules, proteins, peptides, peptidomimetics, glycoproteins, proteoglycans, lipids, phospholipids, lipopolysaccharides, glycolipids, nucleic acids, proteoglycans, carbohydrates. In some embodiments, the biological agent functional component may be anti-neoplastic, anti-microbial, a hormone, an effector. Suitable functional components include well known therapeutic compounds such as anti-neoplastic agents include paclitaxel, daunorubicin, doxorubicin, carminomycin, 4'-epiadriamycin, 4-demethoxy-daunomycin, 11 - deoxydaunorubicin, 13-deoxydaunorubicin, adriamycin-14-benzoate, adriamycin-14-octanoate, adriamycin-14-naphthaleneacetate, vinblastine, vincristine, mitomycin C, N-methyl mitomycin C, bleomycin A2, dideazatetrahydrofolic acid, aminopterin, methotrexate, cholchicine and cisplatin. Suitable anti-microbial agent functional components include aminoglycosides including gentamicin, antiviral compounds such as rifampicin, 3'-azido-3'-deoxythymidine (AZT) and acylovir, antifungal agents such as azoles including fluconazole, plyre macrolides such as amphotericin B, and candicidin, anti-parasitic compounds such as antimonials. Suitable hormone functional components may include toxins such as diphtheria toxin, cytokine such as CSF, GSF, GMCSF, TNF, erythropoietin, immunomodulators or cytokines such as the interferons or interleukins, a neuropeptide, reproductive hormone such as HGH, FSH, or LH, thyroid hormone, neurotransmitters such as acetylcholine, hormone receptors such as the estrogen receptor. Suitable functional components also include non-steroidal anti-inflammatories such as indomethacin, salicylic acid acetate, ibuprofen, sulindac, piroxicam, and naproxen, and anesthetics or analgesics. In some embodiments, the functional component is not a radioisotope.

The other functional components can be naturally occurring or synthetic and may be biologically active in their native state where they can act, for example, at the surface of a target cell or can be transported into the target cell to act intracellularly.

The other functional component can also be an "antibody" which as used herein includes immunoglobulins, which are the product of B cells and variants thereof as well as the T cell receptor (TcR), which is the product of T cells and variants thereof. An immunoglobulin is a protein comprising one or more polypeptides substantially encoded by the immunoglobulin kappa and lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Also subclasses of the heavy chain are known. For example, IgG heavy chains in humans can be any of IgG1, IgG2, IgG3 and IgG4 subclass.

A typical immunoglobulin structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist as full length intact antibodies or as a number of well-characterized fragments produced by digestion with various peptidases or chemicals. Thus, for example, pepsin digests an antibody below the disulfide linkages in the hinge region to produce F(ab')₂, a dimer of Fab which itself is a light chain joined to V_{H}-CH₁ by a disulfide bond. The F(ab')₂ may be reduced under mild conditions to break the disulfide linkage in the hinge region thereby converting the F(ab')₂ dimer into an Fab' monomer. The Fab' monomer is essentially a Fab fragment with part of the hinge region (see, Fundamental Immunology, W. E. Paul, ed., Raven Press, N.Y. (1993), for a more detailed description of other antibody fragments). While various antibody fragments are defined in terms of the digestion of an intact antibody, one of skill will appreciate that any of a variety of antibody fragments may be synthesized de novo either chemically or by utilizing recombinant DNA methodology. Thus, the term antibody, as used herein also includes antibody fragments either produced by the modification of whole antibodies or synthesized de novo or antibodies and fragments obtained by using recombinant DNA methodologies.

The T cell receptor (TcR) is a disulfide linked heterodimer composed of α or β chains or , on a minority of T cells, γ or δ chains. The two chains are generally disulfide-bonded just outside the T cell plasma membrane in a short extended stretch of amino acids resembling the antibody hinge region. Each TcR chain is composed of one Antibody-like variable domain (Vα or Vβ) and one constant domain (Cα or Cβ). The full TcR has a molecular mass of about 95 kDa with the individual chains varying in size from 35 to 47 kDa. Also encompassed within the meaning of TCR are portions of the receptor such as the variable regions of this receptor that can be produced as a soluble protein using methods well known in the art. For example, U.S. Patent No. 6,080,840 describes a soluble T cell receptor (TcR) prepared by splicing the extracellular domains of a TcR to the glycosyl phosphatidylinositol (GPI) membrane anchor sequences of Thy-1. The molecule is expressed in the absence of CD3 on the cell surface, and can be cleaved from the membrane by treatment with phosphatidylinositol specific phospholipase C (PI-PLC). The soluble TcR also may be prepared by coupling the TcR variable domains to an antibody heavy chain CH₂ or CH₃ domain, essentially as described in U.S. Patent No. 5,216,132 or as soluble TcR single chains as described by Schusta et al. Nature Biotech. 18,754-759 (2000) or Holler et al. Proc. Natl. Acad. Sci (USA) 97:5387-5392 (2000). The TcR "antibodies" as soluble products may be used in place of antibody for making the compounds of the invention. The combining site of the TcR can be identified by reference to CDR regions and other framework residues using the same methods discussed above for antibodies.

Recombinant antibodies may be conventional full length antibodies, antibody fragments known from proteolytic digestion, unique antibody fragments such as Fv or single chain Fv (scFv), domain deleted antibodies, and the like. Fragments may include a domains or polypeptides with as little as one or a few amino acid deleted or mutated while more extensive deletion is possible such as deletion of one or more domains.

An Fv antibody is about 50 Kd in size and comprises the variable regions of the light and heavy chain. A single chain Fv ("scFv") polypeptide is a covalently linked V_{H}::V_{L} heterodimer which may be expressed from a nucleic acid including V_{H}- and V_{L}-encoding sequences either joined directly or joined by a peptide-encoding linker. See Huston, et al. (1988) Proc. Nat. Acad. Sci. USA, 85:5879-5883. A number of structures for converting the naturally aggregated, but chemically separated light and heavy polypeptide chains from an antibody V region into an scFv molecule which will fold into a three dimensional structure substantially similar to the structure of an antigen-binding site. See, e.g. U.S. Patent Nos. 5,091,513, 5,132,405 and 4,956,778.

An exemplary the other functional component which is an antibody is one that recognizes and binds to the target of the targeting component. Where the target is an integrin, exemplary and preferred such antibodies are LM609 and its humanized form known as Vitaxin (See, e.g. Publications WO 89/05155 and WO 01/30393.

In an integrin targeting compound of the invention, the at least one targeting component is covalently linked via the linker to one combining site of the antibody. The combining site refers to the part of an antibody molecule that participates in antigen binding. The antigen binding site is formed by amino acid residues of the N-terminal variable ("V") regions of the heavy ("H") and light ("L") chains. The antibody variable regions comprise three highly divergent stretches referred to as "hypervariable regions" or "complementarity determining regions" (CDRs) which are interposed between more conserved flanking stretches known as "framework regions" (FRs). In an antibody molecule, the three hypervariable regions of a light chain (LCDR1, LCDR2, and LCDR3) and the three hypervariable regions of a heavy chain (HCDR1, HCDR2 and HCDR3) are disposed relative to each other in three dimensional space to form an antigen binding surface or pocket. The antibody combining site therefore represents the amino acids that make up the CDRs of an antibody and any framework residues that make up the binding site pocket.

The identity of the amino acid residues in a particular antibody that make up the combining site can be determined using methods well known in the art. For example, antibody CDRs may be identified as the hypervariable regions originally defined by Kabat et al. (see, "Sequences of Proteins of Immunological Interest," E. Kabat et al., U.S. Department of Health and Human Services; Johnson, G and Wu, TT (2001) Kabat Database and its applications: future directions. Nucleic Acids Research, 29: 205-206; http://immuno.bme.nwa.edu). The positions of the CDRs may also be identified as the structural loop structures originally described by Chothia and others, (see Chothia and Lesk, J. Mol. Biol. 196, 901 (1987), Chothia et al., Nature 342, 877 (1989), and Tramontano et al., J. Mol. Biol. 215, 175 (1990)). Other methods include the "AbM definition" which is a compromise between Kabat and Chothia and is derived using Oxford Molecular's AbM antibody modeling software (now Accelrys) or the "contact definition" of CDRs by Macallum et al., ("Antibody-antigen interactions: contact analysis and binding site topography," J Mol Biol. 1996 Oct 11;262(5):732-45). The following chart identifies CDRs based upon various known definitions.

| Loop | Kabat | AbM | Chothia | Contact |
|---|---|---|---|---|
| L1 | L24 -- L34 | L24 -- L34 | L24 -- L34 | L30 -- L36 |
| L2 | L50 -- L56 | L50 -- L56 | L50 -- L56 | L46 -- L55 |
| L3 | L89 -- L97 | L89 -- L97 | L89 -- L97 | L89 -- L96 |
| H1 | H31 -- H35B | H26 -- H35B | H26 -- H32..34 | H30 -- H35B |
| (Kabat Numbering) | | | | |
| H1 | H31 -- H35 | H26 -- H35 | H26 -- H32 | H30 -- H35 |
| (Chothia Numbering) | | | | |
| H2 | H50 -- H65 | H50 -- H58 | H52 -- H56 | H47 -- H58 |
| H3 | H95 -- H102 | H95 -- H102 | H95 -- H102 | H93 -- H101 |

General guidelines by which one may identify the CDRs in an antibody from sequence alone are as follows:
LCDR1:
   Start - Approximately residue 24.
   Residue before is always a Cys.
   Residue after is always a Trp. Typically TRP is followed with TYR-GLN, but also may be followed by LEU-GLN, PHE-GLN, or TYR-LEU.
   Length is 10 to 17 residues.
LCDR2:
   Start - 16 residues after the end of L1.
   Sequence before is generally ILE-TYR, but also may be VAL-TYR, ILE-LYS, or ILE-PHE. Length is generally 7 residues.
LCDR3:
   Start - generally 33 residues after end of L2.
   Residue before is a Cys.
   Sequence after is PHE-GLY-X-GLY.
   Length is 7 to 11 residues.
HCDR1:
   Start - at approximately residue 26 (four residues after a CYS) [Chothia / AbM definition] Kabat definition starts 5 residues later.
   Sequence before is CYS-X-X-X.
   Residues after is a TRP, typically followed by VAL, but also followed by ILE, or ALA.
   Length is 10 to 12 residues under AbM definition while Chothia definition excludes the last 4 residues.
HCDR2:
   Start - 15 residues after the end of Kabat /AbM definition of CDR-H1.
   Sequence before typically LEU-GLU-TRP-ILE-GLY (SEQ ID NO: 1), but a number of variations are possible.
   Sequence after is LYS/ARG-LEU/ILE/VAL/PHE/THR/ALA-THR/SER/ILE/ALA
   Length is 16 to 19 residues under Kabat definition (AbM definition ends 7 residues earlier).
HCDR3:
   Start -33 residues after end of CDR-H2 (two residues after a CYS).
   Sequence before is CYS-X-X (typically CYS-ALA-ARG).
   Sequence after is TRP-GLY-X-GLY.
   Length is 3 to 25 residues.

The identity of the amino acid residues in a particular antibody that are outside the CDRs, but nonetheless make up part of the combining site by having a side chain that is part of the lining of the combining site (i.e., it is available to linkage through the combining site), can be determined using methods well known in the art such as molecular modeling and X-ray crystallography. See e.g., Riechmann et al., (1988) Nature, 332:;323-327. The aldolase antibody mouse mAb 38C2, which has a reactive lysine near to but outside HCDR3, is an example of such an antibody.

The reactive residue of the antibody combining site may be naturally associated with the antibody such as when the residue is encoded by nucleic acid present in the lymphoid cell first identified to make the antibody. Alternatively, the amino acid residue may arise by purposely mutating so as to encode the particular residue (see, e.g., WO 01/22922 to Meares et al.). In another approach, the amino acid residue or its reactive elements (e.g., a nucleophilic amino group or sulfhydryl group) may be attached to an amino acid residue in the antibody combining site. Thus, covalent linkage with the antibody occurring "through an amino acid residue in the combining site of the antibody" as used herein means that linkage can be made directly to an amino acid residue of an antibody combining site or indirectly through a chemical moiety that is linked to a side chain of an amino acid residue of an antibody combining site.

Functional components that are proteins including the aldolase antibody can be linked to targeting components via a reactive side chain in the protein. The reactive side chain may be present or may arise by mutation. The reactive side chain in lysine (epsilon amino group) may be covalently linked to a linker comprising a ketone, diketone, beta lactam, active ester haloketone, lactone, anhydride, maleimide, epoxide, aldehyde amidine, guanidine, imines, eneamines, phosphates, phosphonates, epoxides, aziridines, thioepoxides, masked or protected diketones (ketals for example), lactams, haloketones, aldehydes, and the like. Such a reactive lysine side chain is present in the combining site of the aldolase antibody e.g., mouse monoclonal antibody mAb 38C2 and other like catalytic antibodies as well as suitably humanized and chimeric versions of such antibodies. Mouse mAb 38C2 is the prototype of a new class of catalytic antibodies that were generated by reactive immunization and mechanistically mimic natural aldolase enzymes (Barbas et al., 1997, Science 278, 2085-2092). Through a reactive lysine, these antibodies catalyze aldol and retro-aldol reactions using the enamine mechanism of natural aldolases (Wagner et al., 1995, Science 270, 1797-1800; Barbas et al., 1997, Science 278, 2085-2092; Zhong et al., 1999, Angew. Chem. Int. Ed. 38, 3738-3741; Karlstrom et al., 2000, Proc. Natl. Acad. Sci. U.S.A., 973878-3883). In addition to their versatility and efficacy in synthetic organic chemistry (e.g., Hoffmann et al., 1998, J. Am. Chem. Soc. 120, 2768-2779 ; Sinha et al., 1998, Proc. Natl. Acad. Sci. U.S.A. 95, 14603-14608), aldolase antibodies have been used to activate camptothecin, doxorubicin, and etoposide prodrugs in vitro and in vivo as an anti-cancer strategy (Shabat et al.,1999, Proc. Natl. Acad. Sci. U.S.A. 96, 6925-6930 and ,2001, Proc. Natl. Acad. Sci. U.S.A. 98, 7528-7533).

The reactive amino acid of a functional component such as the aldolase antibody may be a reactive cysteine, serine or tyrosine residue. For cysteines, the resulting antibody may form a covalent linkage with maleimide-containing components or other thiol-reactive groups such as iodoacetamides, aryl halides, disulfhydryls and the like. Reactive cysteines may be found in thioesterase catalytic antibodies as described by Janda et al., Proc. Natl. Acad. Sci. (USA) 91:2532-2536, (1994). For other esterase antibodies, see Wirsching et al., Science 270:1775-82 (1995). Reactive amino acid-containing functional components may be prepared by means well known in the art including mutating the amino acid residue to encode for the reactive amino acid or chemically derivatizing an amino acid side chain that contains the reactive group.

The components of the targeting compound are linked preferably covalently and preferably with a linker or branched linker. In some embodiments where an antibody is the functional component, the targeting component may be covalently linked to the antibody combining site by using a linker. An appropriate linker can be chosen to provide sufficient distance between the targeting component and the functional component in order for the targeting component to be able to bind to its target molecule. Where linkage to the combining site of an antibody is desired, an appropriate linker can be chosen to provide sufficient distance between the targeting component and the antibody combining site in order for the targeting component to be able to bind to its target molecule. This distance depends on several factors including, for example, the distance from the outermost surface of the antibody combining site to the reactive side chain in the combining site, and the nature of the targeting agent. Generally, the linker will be between about 5 to 10 angstroms (0.5 to 1 nm) in length, with 10 or more angstroms (1.0 nm) being more preferred, although shorter linkers of about 3 angstroms (0.3 nm) in length may be sufficient if the amino acid side chain is very near to the outermost portion of the combining site and/or the targeting component includes a segment that can function as a part of a linker.

Linker length may also be viewed in terms of the number of linear atoms (cyclic moieties such as aromatic rings and the like to be counted by taking the shortest route). Linker length under this measure is generally about 10 to 200 atoms and more typically about 30 or more atoms, although shorter linkers of two or more atoms may be sufficient if the reactive amino acid side chain is very near to the outermost portion of the antibody combining site. Generally, linkers with a linear stretch of at least about 9 atoms are sufficient. The above linker lengths for linking to antibody combining sites are generally applicable to linking integrin targeting components to non-antibody functional components.

Other linker considerations include the effect of the linker on physical or pharmacokinetic properties of the resulting targeting compound such as, solubility, lipophilicity, hydrophilicity, hydrophobicity, stability (more or less stable as well as planned degradation), rigidity, flexibility, immunogenicity, modulation of binding, chemical compatibility with targeting agent, ability to be incorporated into a micelle or liposome, and the like. For the RGD peptidomimetic targeting components of the prevention, the linker may be attached to the spacer between the core of the molecule and the basic or acid group. Alternatively, the linker can be attached to the core itself.

In some embodiments, the linker includes any atom from the group C, H, N, O P, S, Si, halogen (F, Cl, Br, I) or a salt thereof. The linker also may include a group such as an alkyl, alkenyl, alkynyl, oxoalkyl, oxoalkenyl, oxoalkynyl, aminoalkyl, aminoalkenyl, aminoalkynyl, sulfoalkyl, sulfoalkenyl, or sulfoalkynyl group, phosphoalkyl, phosphoalkenyl, phosphoalkynyl group, as well as a carbocyclic or heterocyclic mono or fused saturated or unsaturated ring structure. Combinations of the above groups and rings may also be present in the linkers of the targeting compounds of the invention.

The general design of a embodiment of a linear linker for use in targeting compounds of the present invention is shown in FIG. 2. The linker includes three functional areas identified starting from the targeting agent (T), being the connecting chain (X), the recognition group (Y) and the reactive group (Z). The integrin targeting agent-linker compound SCS-873 is shown in FIG. 2 with the linker portions X, Y and Z identified. In some embodiments, the recognition group may not be needed.

Linker reactive group Z may include one or more C=O, groups arranged to form a diketone, an acyl beta-lactam, an active ester, haloketone, a cyclohexyl diketone group, an aldehyde or maleimide. Other groups may include lactone, anhydride, and alpha-haloacetamide and epoxide. Exemplary linker electrophilic reactive groups that can covalently bond to a reactive nucleophilic group (e.g. lysine or cysteine side chain) of a functional component or integrin targeting component include acyl beta-lactam, simple diketone, succinimide active ester, maleimide, haloacetamide with linker, haloketone, cyclohexyl diketone, aldehyde, amidine, guanidine, imine, eneamine, phosphate, phosphonate, epoxide, aziridine, thioepoxide, sulfonate, a masked or protected diketone (a ketal for example), lactam, and the like, masked C=O groups such as imine, ketal, acetal and any other known electrophilic group. A preferred linker reactive group includes one or more C=O, groups arranged to form a acyl beta-lactam, simple diketone, succinimide active ester, maleimide, haloacetamide with linker, haloketone, cyclohexyl diketone, or aldehyde.

Linker diketone reactive groups which form reversible covalent bonds with the reactive lysine or cysteine of a functional component or integrin targeting component are shown in FIG. 6. R₁ and R₂ and R₃ in structures A-C represent substituents which can be C, H, N, O P, S, Si, halogen (F, Cl, Br, I) or a salt thereof. These substituents may also include a group such as an alkyl, alkenyl, alkynyl, oxoalkyl, oxoalkenyl, oxoalkynyl, aminoalkyl, aminoalkenyl, aminoalkynyl, sulfoalkyl, sulfoalkenyl, or sulfoalkynyl group, phosphoalkyl, phosphoalkenyl, phosphoalkynyl group. R₂ and R₃ could be cyclic as exemplified in structures B and C while X could be a heteroatom. Other diketone linker reactive groups are shown in FIG. 7 as structures B and G. FIG. 7 also includes the structures of other preferred linker reactive groups.

In some embodiments, the linker may include a reactive group that forms a nonreversible covalent bond with the combining site of an antibody. Exemplary such reactive groups, shown as structures D-G in FIG. 6, are useful for nonreversibly attaching a targeting component-linker to a reactive nucleophilic group (e.g. lysine or cysteine side chain) in the functional component (or vice versa). Other diketone linker reactive groups that form nonreversible covalent bonds are shown in FIG. 7 as structures A, C and D.

Linkers may optionally contain a recognition group Y situated between the reactive group portion and the connecting chain portion of the linker such as shown in FIG. 2. While not wishing to be bound by any theory, the recognition group, if present, may work to properly position the reactive group into a binding site such an antibody combining site so that it may react with a reactive amino acid side chain. FIG. 8 shows a variety of exemplary recognition groups with one or more homo or hetero ring structures of five or six atoms. Larger ring structures are also possible.

Various embodiments of the connecting chain X portion of the general linker design (FIG. 2) is shown in FIG. 9. As shown, the connecting chain may vary considerably in length with both straight chain and branched chain structures possible.

A preferred linker for use in targeting compounds of the invention and for preparing targeting agent-linker compounds or functional component-linker compounds is a linker with a 1,3-diketone reactive group have the structure 33 as shown below where n is from 1-100 or more and preferably is 1, 2, or 4, and more preferably is 3. In some embodiments, the linker is a repeating polymer such as polyethylene glycol.

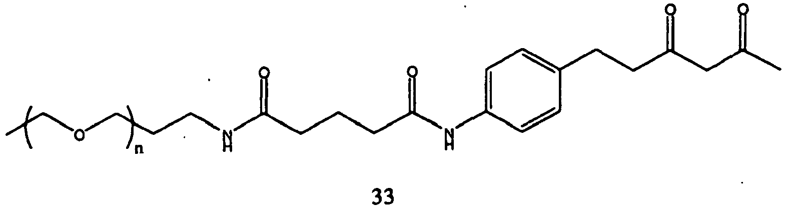

The linker reactive group or similar such reactive group that may be inherent in the targeting component can be chosen for linkage with a particular functional component. For example, a chemical moiety for modification by an aldolase antibody may be a ketone, diketone, beta lactam, active ester haloketone, lactone, anhydride, maleimide, alpha-haloacetamide, cyclohexyl diketone, epoxide, aldehyde, amidine, guanidine, imine, eneamine, phosphate, phosphonate, epoxide, aziridine, thioepoxide, masked or protected diketone (ketal for example), lactam, haloketone, aldehyde, and the like. A 1,3-diketone configuration such as the diketone shown in Compound SCS-873 (see below) or SCS-864 (see below), is especially preferred as a substrate for modification by an aldolase antibody.

A linker reactive group chemical moiety suitable for covalent modification by a reactive sulfhydryl group of a functional component or integrin targeting component may be a disulfide, aryl halide, maleimide, alpha-haloacetamide, isocyanate, epoxide, thioester, active ester, amidine, guanidine, imine, eneamine, phosphate, phosphonate, epoxide, aziridine, thioepoxide, masked or protected diketone (ketal for example), lactam, haloketone, aldehyde. One of skill in the art will readily appreciate that reactive amino acid side chains in protein functional components may possess an electrophilic group that reacts with a nucleophilic group on the targeting component or its linker, whereas in other embodiments a reactive nucleophilic group in an amino acid side chain of a protein functional group reacts with an electrophilic group in a targeting component or linker. Thus, protein component side chains may be substituted with an electrophile (e.g., FIGs. 6 and 7) and this group may be used to react with a nucleophile on the targeting component or its linker (e.g., NH₂). In this embodiment, the functional and targeting components each have a partial linker with appropriate reactive moieties at each end so that the two ends of the partial linker can form the full linker, thus creating the complete targeting compound.

Integrin targeting compounds may be prepared by several approaches. In one approach, a targeting component-linker compound is synthesized with a linker that includes one or more reactive groups designed for covalent reaction with a susceptible reactive moiety on the functional component. In a preferred embodiment, the suitable reactive moiety can be a side chain of an amino acid. The component-linker compound and functional component are then combined under conditions where the linker reactive group forms a covalent bond with the functional component. "Susceptible" as used herein with reference to a chemical moiety indicates that the chemical moiety will covalently bond with a compatible reactive group. Thus, an electrophilic group is susceptible to covalent bonding with a nucleophillic group and vice versa.

In another approach, linking can be achieved by synthesizing a functional component-linker compound comprising the functional component and a linker where the linker includes one or more reactive groups designed for covalent reaction with a susceptibe chemical moiety of the targeting component. The targeting component may need to be modified to provide the appropriate reactive moiety for reaction with the linker reactive group. The functional component-linker and targeting component are combined under conditions where the linker reactive group covalently links to the targeting component.

A further approach for forming targeting compounds of the invention uses a dual linker design. In one embodiment, a targeting component-linker compound is synthesized which comprises a targeting component and a linker with a reactive group. A functional component-linker compound also is synthesized which comprises a functional component and a linker, the latter with a chemical moiety susceptible to reactivity with the reactive group of the component-linker of the first step. These two linker containing compounds are then combined under conditions whereby the linkers covalently link, forming the targeting compound.

In yet another embodiment, a functional component-linker compound is synthesized which comprises a functional component and a linker with a reactive group. A targeting component-linker compound is also prepared which comprises the component and a linker, the latter with a chemical moiety susceptible to reactivity with the reactive group of the antibody-linker of the first step. These two linker containing compounds are then combined under conditions whereby the linkers covalently link, forming the targeting compound.

Numerous means well known in the art can be used to attach a linker to the targeting agent or antibody combining site. Exemplary functional groups that can be involved in the linkage include, for example, esters, amides, ethers, phosphates, amino, keto, amidine, guanidine, imines, eneamines, phosphates, phosphonates, epoxides, aziridines, thioepoxides, masked or protected diketones (ketals for example), lactams, haloketones, aldehydes, thiocarbamate, thioamide, thioester, sulfide, disulfide, phosphoramide, sulfonamide, urea, thioruea, carbamate, carbonate, hydroxamide.

A functional component can be linked to a targeting component using a linker moiety that is labile under certain conditions. The labile linkage may be between the functional component and the linker, between the targeting component and the linker, or within the linker, or combinations thereof. For example, the linker may be labile when subjected to a certain pH. The linker may also be a substrate for a particular enzyme, such as an enzyme present in body fluids. Thus, the particular design of the labile linker may be used to direct the release of the biological agent functional component after it has reached it intended target. A labile linker may be a reversibly covalent bond. Such linker may be an acid-labile linker such as a cis-aconitic acid linker that takes advantage of the acidic environment of different intracellular compartments such as the endosomes encountered during receptor mediated endocytosis and the lysosomes. See Shen et al., Biochem. Biophys. Res. Commun. (1981) 102:1048-1054; Yang et al., J. Natl. Canc. Inst. (1988) 80: 1154-1159. In other embodiments, a peptide spacer arm is employed as the linker so that the functional component can be released by the action of a peptidase such as a lysosomal peptidase. See e.g., Trouet et al., Proc. Natl. Acad. Sci. (1982) 79: 626-629. Shown below (Compound 34) is an exemplary targeting compound of this invention wherein the targeting moiety is an integrin antagonist and the functional component is propyrrolinodoxorubicin (R=peptides) and the two are linked by a pH sensitive labile linker.

Labile linkers include, reversible covalent bonds, pH sensitive linkages (acid or base sensitive), enzyme sensitive linkages, degradation sensitive linkers, photosensitive linkers, and combinations thereof. These features are also characteristic of a prodrug which can be considered as a type of labile linker. A variety of labile linkers have been previously moieties designed. For example, prodrugs can be formed using compounds having carboxylic acid that slowly degrade by hydrolysis as described in U.S. Patent No. 5,498,729.

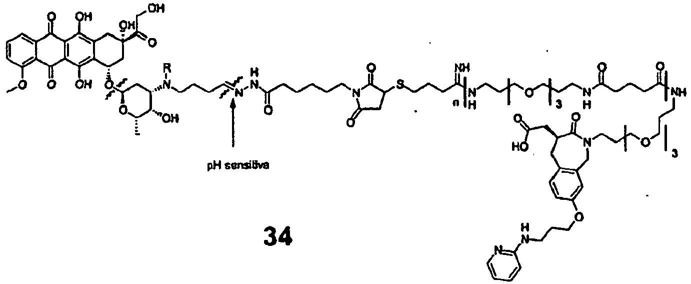

In this regard, the other functional component can be a "prodrug," meaning that the functional component is essentially therapeutically inactive, but becomes active upon some modification. The prodrug can be delivered at the surface of a cell or intracellulary using antibody targeting compounds of the invention where it can then be activated. In the prodrug approach, site-specific drug delivery can be obtained from tissue-specific activation of a prodrug, which is the result of metabolism by an enzyme that is either unique for the tissue or present at a higher concentration (compared with other tissues); thus, it activates the prodrug more efficiently.

Photodynamic treatment may be used to activate a prodrug by cleaving a photosenitive linker or by activating a photoresponsive enzyme (acyl enzyme hydrolysis) as described previously (see U.S. Patent No. 5,114,851 and 5,218,137). Photodynamic treatment also may be used to rapidly inactivate a drug in sites where the drug activity is not desired (e.g. in non-target tissues). Various means of covalently modifying a drug to form a prodrug are well known in the art.

One desiring to make a compound of the present invention simply selects as the other functional component a structure having a pre-selected activity. In one embodiment, the functional component is a therapeutic agent such as drug. Any suitable drug can be used. Selection of the therapeutic agent depends upon the desired activity and target of the present compound. Where the target is an integrin, a preferred therapeutic is an agent having biological activity directed against the integrin. For example, in the case of Kaposi's Sarcoma, a cancer associated with angiogenesis of cancerous lesions, one can chose any of various drugs such as, for example, the three drugs paclitaxel, doxorubicin, and etoposide with demonstrated therapeutic efficacy in this disease. A derivative of doxorubicin, 2-pyrrolinodoxorubicin is 500-1000 times more potent than doxorubicin itself and it has been extensively studied in other drug targeting strategies (for a recent review see Schally and Nagy, Eur. J. Endocrinology 141, 1-14, 1999). A compound comprising an integrin targeting component with any of these compounds can be used to treat the abnormal angiogenesis of Kaposi's Sarcoma. Synthesis of these compounds with the integrin targeting component SCS-873 is described in the Examples.

The integrin targeting compounds of the present invention have many uses. In one approach, a functional component can be delivered to integrin associated with cells, tissue or fluid macromolecule of an individual by administering the targeting compound. In one approach, the other functional component is a therapeutic agent.

The integrin targeting invention compounds have particular utility for treating a pathological condition associated with integrin expression. Accordingly, there is provided an integrin targeting compound of the invention for use in the treatment of a disease or condition in an individual wherein said disease or condition involves an integrin, by administering to the individual a therapeutically effective amount of an invention targeting compound comprising a therapeutic component effective against the disease or condition. In one such embodiment, the condition is a carcinoma. The association of integrin expression in carcinomas is well known in the art (See, e.g., U.S. Pat. Nos. 5,753,230 and 5,766,591).

The integrin targeting compounds of the present invention may be used for treating any disease or condition that is associated with the integrin being targeted. For example, the vitronectin receptor on osteoclasts is known to inhibits osteoclastic bone resorption. Thus, diseases or conditions in which bone resorption is associated with a pathology, such as osteoporosis and osteoarthritis, can be treated by administering a vitronectin targeting compound of the invention. Alternatively, a vitronectin targeting compound with an appropriate functional component can be used to stimulate bone formation by increasing osteocalcin release by osteoclasts. Increased bone production is a clear benefit in disease states wherein there is a deficiency of mineralized bone mass or remodeling of bone is desired, such as fracture healing and the prevention of bone fractures. Diseases and metabolic disorders which result in loss of bone structure would also benefit from such treatment. For instance, hyperparathyroidism, Paget's disease, hypercalcemia of malignancy, osteolytic lesions produced by bone metastasis, bone loss due to immobilization or sex hormone deficiency, Behcet's disease, osteomalacia, hyperostosis and osteopetrosis, could benefit from administering a compound of this invention.

The integrin targeting compounds of the present invention also may be used for treating any inflammatory disorders, such as rheumatoid arthritis and psoriasis, and cardiovascular diseases, such as atherosclerosis and restenosis, which involve vitronectin expressing cells. Accordingly, vitronectin targeting compounds of the invention which comprise an appropriate functional component can be used to treat these disorders. This approach also applies to the treatment or prevention of other diseases including, but not limited to, thromboembolic disorders, asthma, allergies, adult respiratory distress syndrome, graft versus host disease, organ transplant rejection, septic shock, eczema, contact dermatitis, inflammatory bowel disease, and other autoimmune diseases. The compounds of the present invention may also be useful for wound healing.

The integrin targeting compounds of the present invention further find use in treating angiogenic disorders. Such disorders involve abnormal neovascularization where growth of new blood vessels is the cause of, or contributes to, the pathology associated with a disease. In these situations, inhibition of angiogenesis will reduce the deleterious effects of the disease. Other therapeutic targets for the compounds of the instant invention are eye diseases characterized by neovascularization. Such eye diseases include corneal neovascular disorders, such as corneal transplantation, herpetic keratitis, luetic keratitis, pterygium and neovascular pannus associated with contact lens use. Additional eye diseases include age-related macular degeneration, presumed ocular histoplasmosis, retinopathy of prematurity, neovascular glaucoma,

Where the growth of new blood vessels is required to support growth of a deleterious tissue, inhibition of angiogenisis will reduce the blood supply to the tissue and thereby contribute to reduction in tissue mass based on blood supply requirements. Cancer is an example where neovascularization is a continual requirement in order for the tumor to grow and establish tumor metastases. Thus, the integrin targeting compounds of the present invention inhibit tumor tissue angiogenesis, thereby preventing tumor metastasis and tumor growth.

In addition to therapeutic applications, integrin targeting compounds of the invention also may be used for imaging of cells or tissues such as tumor cells as is well known in the art. Accordingly, provided is a method of imaging cells or tissue in an individual wherein said cells or tissue expresses an integrin target molecule, said method comprising administering to the individual an effective amount of the integrin targeting compound linked to a suitable radioisotope or detectable label. The radioisotope or label may be attached to the targeting component or the functional component.

An integrin targeting compound of the present invention can be administered as a pharmaceutical composition wherein the invention compound is formulated with a pharmaceutically acceptable carrier. Accordingly, the invention compounds may be used in the manufacture of a medicament. Pharmaceutical compositions of the invention compounds may be formulated as solutions or lyophilized powders for parenteral administration. Powders may be reconstituted by addition of a suitable diluent or other pharmaceutically acceptable carrier prior to use. Powders also may be sprayed in dry form. The liquid formulation may be a buffered, isotonic, aqueous solution. Examples of suitable diluents are normal isotonic saline solution, standard 5% dextrose in water or buffered sodium or ammonium acetate solution. Such formulation is especially suitable for parenteral administration, but may also be used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride or sodium citrate.

Alternately, integrin targeting compounds may be encapsulated, tableted or prepared in a emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar or gelatin. Liquid carriers include syrup, peanut oil, olive oil, saline and water. The carrier may also include a sustained release material such as glyceryl monostearate or glyceryl distearate, alone or with a wax. The amount of solid carrier varies but, preferably, will be between about 20 mg to about 1 g per dosage unit. The pharmaceutical preparations are made following the conventional techniques of pharmacy involving milling, mixing, granulating, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. For rectal administration, the invention compounds may be combined with excipients such as cocoa butter, glycerin, gelatin or polyethylene glycols and molded into a suppository.

Integrin targeting compounds of the invention may be formulated to include other medically useful drugs or biological agents. The compounds also may be administered in conjunction with the administration of other drugs or biological agents useful for the disease or condition that the invention compounds are directed (see e.g., U.S. Pat. No. 6,413,955 for active ingredients useful for osteoporosis).

As employed herein, the phrase "an effective amount," refers to a dose sufficient to provide concentrations high enough to impart a beneficial effect on the recipient thereof. The specific therapeutically effective dose level for any particular subject will depend upon a variety of factors including the disorder being treated, the severity of the disorder, the activity of the specific compound, the route of administration, the rate of clearance of the compound, the duration of treatment, the drugs used in combination or coincident with the compound, the age, body weight, sex, diet and general health of the subject, and factors well known in the medical arts and sciences. Various general considerations taken into account in determining the "therapeutically effective amount" are known to those of skill in the art and are described, e.g., in Gilman et al., eds., Goodman And Gilman's: The Pharmacological Bases of Therapeutics, 8th ed., Pergamon Press, 1990; and Remington's Pharmaceutical Sciences, 17th ed., Mack Publishing Co., Easton, Pa., 1990. Dosage levels typically fall in the range of about 0.001 up to 100 mg/kg/day; with levels in the range of about 0.05 up to 10 mg/kg/day are generally applicable. A compound can be administered parenterally, such as intravascularly, intravenously, intraarterially, intramuscularly, subcutaneously. Administration can also be orally, nasally, rectally, transdermally or inhalationally via an aerosol. The composition may be administered as a bolus, or slowly infused.

The administration of the targeting compound to an immunocompetent individual may result in the production of antibodies against the compound. Such antibodies may be directed to the targeting component, the functional component or any other entity associated with the compound. The immunogenicity of such compound may be addressed by methods well known in the art such as by attaching long chain polyethylene glycol (PEG)-based spacers, and the like, to one or more components of the compound. Long chain PEG and other polymers are known for their ability to mask foreign epitopes, resulting in the reduced immunogenicity of therapeutic proteins that display foreign epitopes (Katre et al., 1990, J. Immunol. 144, 209-213; Francis et al., 1998, Int. J. Hematol. 68, 1-18). As noted, PEG can serve as a linker in targeting compounds of the invention, thus providing both linker function and reduced immunogenicity. Alternatively or in addition, the individual may be administered an immunosuppressent drug such as cyclosporin A, anti-CD3 antibody, and the like, to reduce the likelihood that an immune response to the targeting compound will develop.

The RGD peptidomimetic can be used to target liposomes. This is achieved by chemically linking such targeting components to a lipid moiety that enables the targeting component to associate with lipids of the liposome. The liposome encapsulated with an appropriate drug can then be targeted more effectively in vivo by aid of the targeting component. For example, treatment of Kaposi's sarcoma with sterically stabilized stealth liposomes containing doxorubicin (DoxilTM) is one of the most effective of the approved KS therapies. This liposomal formulation provides several advantages over administration of free doxorubicin. In addition to a reduction in cardiac toxicity, vomiting, alopecia, peripheral neuropathy, and mucositis provided by the liposomal formulation, DoxilTM possesses an intrinsic capacity for passive targeting to the tumor as a result of particulate size and the enhanced permeability and retention phenomenon (Matsumura et al., Cancer Res. 46, 6387-6392, 1986) that is further aided by the extended serum half-life of the drug.

Active targeting as disclosed herein can augment the passive targeting capacity of DoxilTM thereby more efficiently delivering the cytotoxic payload of the liposomes to the KS tumor and its vasculature. Such active targeting of DoxilTM likely will reduce the dosage needed for clinical response thereby limiting non-specific toxicities. Administration of targeted liposomes may be achieved as previously described (See, e.g., Allen et al., Adv. Drug Deliv. Rev. 21, 117-133). The targeting compounds of the invention may be used in combination with other compounds or therapies such as radiation therapy.

It would be readily evident that the compounds of the invention find use not only in human medical therapy and diagnosis but also in vitro diagnostics, veterinary, agricultural, environmental and other disciplines. The versatility of the invention is illustrated by the following Examples which illustrate preferred embodiments of the invention and are not limiting of the claims or specification in any way.

### EXAMPLE 1: Antibody targeting compound comprising an RGD peptidomimetic targeting agent covalently linked to the combining site of aldolase monoclonal antibody 38C2.

An integrin targeting compound was formed based on the formation of a reversible covalent bond between a diketone linker derivative of an RGD peptidomimetic and the reactive lysine of mouse mAb 38C2. Mouse mAb 38C2 is the prototype for a new class of catalytic antibodies generated by reactive immunization and mechanistically mimic natural aldolase enzymes (Barbas et al., Science 278, 2085-2092, 1997). Through a reactive lysine, these antibodies catalyze aldol and retro-aldol reactions using the enamine mechanism of natural aldolases (Wagner et al., Science 270, 1797-1800, 1995; Barbas et al., Science 278, 2085-2092, 1997; Zhong et al., Angew. Chem. Int. Ed. 38, 3738-3741, 1999). In addition to their versatility and efficacy in synthetic organic chemistry, aldolase antibodies have been used in the activation of camptothecin, doxorubicin, and etoposide prodrugs *in vitro* and *in vivo* as an anti-cancer strategy (Shabat et al., Proc. Natl. Acad. Sci. U.S.A. 96, 6925-6930, 1999); Shabat, D. et al. Proc. Natl. Acad. Sci. U.S.A. 98, 7528-7533, 2001). Yet another feature of these antibodies, namely their ability to bind diketones covalently, has remained largely unexplored.

The RGD peptidomimetic used (see Compound 1) is specific for human integrin with a high binding affinity for αᵥβ₃ at 0.9 nM and αᵥβ₅ at 0.6 nM (specificity exhibited by minimal a_{llb}b₃ binding) (Miller et al., supra). A diketone linker modified version of Compound 1, designated SCS-873, was prepared as described in Example 3. SCS-873 is shown below with the targeting component and the linker separately identified.

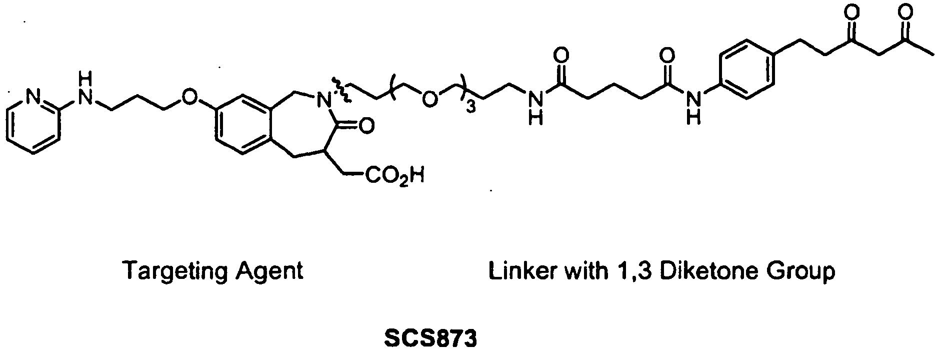

A peptidomimetic RGD antagonist with known activity for both αᵥβ₃ or aᵥβ₅ binding is desirable because some of these compounds bind both murine and human integrins. Such species cross reactivity affords preclinical *in vivo* studies in animal angiogenesis models prior to human trials. In addition, the targeting compound may be used for the therapy of Kaposi's sarcoma which is associated with αᵥβ₃ integrin.

SCS-873 was linked to antibody 38C2 by the following procedure: One milliliter antibody 38C2 in phosphate buffered saline (10mg/ml) was added to 12 microliters of a 10 mg/mL stock solution of SCS-873 and the resulting mixture was maintained at room temperature for 2 hours prior to use.

The binding of a mixture of SCS-873 and 38C2 to SLK cells was evaluated. SCS-873 effectively mediated cell surface binding of 38C2. No binding of 38C2 was detectable in the absence of SCS-873. Control experiments confirmed that the diketone moiety of the linker is required for binding of SCS-873 to 38C2. It was determined that SCS-873 retains the integrin specificity of the integrin targeting component, i.e., no binding to a_{llb}b₃ in ELISA was detected while binding to αᵥβ₃ and αᵥβ₃ was found to be strong. Independent i.p. and i.v. injections of the targeting compound prepared with SCS-873 and 38C2 versus each component alone into mice demonstrated integrin targeting *in vivo.* In these experiments, the serum half-life of SCS-873 was extended by more than two orders of magnitude through binding to 38C2. Free SCS-873 not bound to antibody had a serum half-life of only minutes while the combination of antibody and small molecule could be detected in the serum sampled from eye bleeds after several days.

### EXAMPLE 2: Integrin Targeting with Protein Functional Components.

The integrin targeting component can be covalently attached to a functional component such as a protein in order to channel effector functions triggered by these proteins. Such linking can be achieved by a lysine-reactive N-hydroxysuccinimide or a cysteine-reactive maleimide functionality.

For example, conjugation of the integrin targeting compound to an IgM antibody induces complement-mediated cytotoxicity; conjugation to IL-2 results in cell-mediated cytotoxicity. A variety of antibodies that neutralize growth factors involved in tumor angiogenesis can be modified with the integrin targeting compound in order to increase their selectivity. For example, a monoclonal antibody that neutralizes VEGF is highly selective if conjugated to the integrin targeting compound. Taking the potential immunogenicity of these conjugates into consideration, long chain polyethylene glycol (PEG)-based spacers can be introduced between the protein-reactive and the integrin targeting functionality. Long chain PEG and other polymers are known for their ability to mask foreign epitopes, resulting in the reduced immunogenicity of therapeutic proteins that display foreign epitopes (Katre et al., 1990, J. Immunol. 144, 209-213; Francis et al., 1998, Int. J. Hematol. 68, 1-18).

### EXAMPLE 3: Synthesis of Targeting Components-Linkers Molecules

Integrin targeting components shown as compounds 15 and 4 were synthesized as shown in the FIG. 10 (Scheme 1) and FIG. 11 (Scheme 2), respectively. A linker with a diketone reactive moiety was added to these targeting molecules as shown in Scheme 3 (FIG. 12) to form targeting compound-linker molecules SCS-873 and SCS-1655. Synthesis of **SCS-873** was achieved starting from compound **14** in three steps. Compound 14 was converted to 15 as shown in Scheme 1 and the crude product was reacted with an N-hydroxysuccinimide (NHS) -ester of the diketone compound **23** in CH₃CN-DMF in the presence of Et₃N. Purification over silica gel (CH₂Cl₂-MeOH, 9:1) afforded pure **SCS-873.**

Compound **SCS-1655** was synthesized from **14** in five steps (Schemes 2 and 3). Deprotection of the BOC group in compound **14** followed by reaction with the NHS ester of the bivalent linker **24** afforded compound **25,** which was then deprotected and reacted with **23** as above to afford **SCS-1655.**

Synthesis of integrin targeting component-linker molecules SCS-864 and SCS-789 is shown in Scheme 4 (FIG. 13). SCS-864 and SCS-789 were each synthesized in one step from compound 4 (FIG. 13, scheme 4). Linking of Compound 4 was achieved with the appropriate activated NHS-ester. SCS-864 is shown below with the targeting component and linker separately identified.

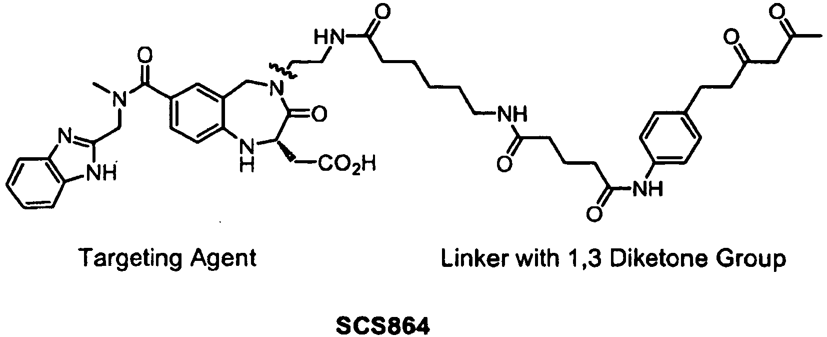

### EXAMPLE 4: Synthesis of an Integrin Targeting Compound with Paclitaxel as the Functional Component (not part of the invention)

Paclitaxel-SCS-873 is synthesized starting from taxol-succinate prepared as previously described (Deutsch et al., J. Med. Chem. 32, 788-792, 1989). Following activation of the carboxy group with PyBOP in DMF, the SCS-amine is directly coupled (Huang et al., Chemistry & Biology 7, 453-461, 2000) providing Paclitaxel-SCS-873. This derivative is analogous to a previously described somatostatin antagonist peptide targeted paclitaxel derivative (Huang et al., Chemistry & Biology 7, 453-461, 2000) that demonstrated good activity and targeting, thereby validating the succinate-based linker strategy for this drug. Others have described paclitaxel prodrugs utilizing a 2'-carbamate instead of a succinate-based linker with success (de Groot et al., J. Medicinal Chem. 43, 3093-3102, 2000). Paclitaxel-SCS-873 is considerably more soluble than paclitaxel itself which suffers from poor solubility. The structure of Paclitaxel-SCS-873 is shown below.

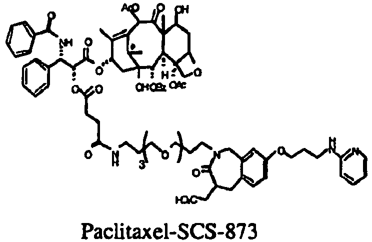

### EXAMPLE 5: Synthesis of an Integrin Targeting Compound with Doxorubicin or 2-Pyrrolinodoxorubicin as the Functional Component (not part of the invention)

Doxorubicin-SCS-873 is prepared using a synthetic scheme similar to that described for doxorubicin derivatives of luteinizing hormone-releasing hormone and somatostatin conjugates (Nagy et al., Proc. Natl. Acad Sci. U.S.A. 93, 7269-7273, 1996; Nagy et al., Proc. Natl. Acad Sci. U.S.A. 95, 1794-1799, 1998). N-Fmoc-DOX-14-O-hemiglutarate are prepared as described by Nagy et al. (Proc Natl. Acad. Sci. 93, 2464-2469, 1996) and subsequently activated with PyBOP in DMF followed by addition of the SCS-amine and removal of the Fmoc protective group affording Doxorubicin-SCS-873. The structure of Doxorubicin-SCS-873 is shown below.

2-Pyrrolinodoxorubicin-SCS-873 can be prepared from Doxorubicin-SCS-873 by reaction with 4-iodobutyraldehyde as described for LH-RH-2-Pyrrolinodoxorubicin conjugates (Nagy et al., Proc Natl. Acad Sci. 93, 2464-2469, 1996). A variety of other synthetic approaches are also available. The proven activity of LH-RH-2-pyrrolinodoxorubicin and doxorubicin conjugates of similar design supports the design of our integrin targeted derivatives (See, e.g., Schally and Nagy, Eur. J. Endocrinology 141, 1-14, 1999 and references therein for a review peptide targeted drug conjugates). The structure of 2-Pyrrolinodoxorubicin-SCS-873 is shown below.

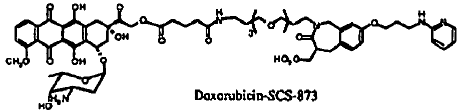

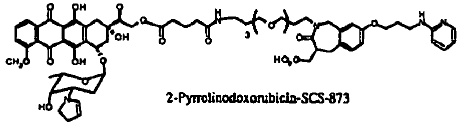

### EXAMPLE 6: Synthesis of an Integrin Targeting Compound with Etoposide as the Functional Component (not part of the invention)

Etoposide-SCS-873 is be prepared from the p-nitrophenylcarbonate recently described for structurally similar etoposide prodrugs that are activated by catalytic antibody (Shabat et al., Proc. Natl. Acad. Sci. U.SA. 98, 7528-7533, 2001). The retro-Michael step was readily catalyzed by endogenous cellular enzymes as well as by the catalytic antibody (Shabat et al., Proc. Natl. Acad. Sci. U.S.A. 96, 6925-6930, 1999). The design of Etoposide-SCS-873 is based on endogenous enzyme or general acid base activation via a retro-Michael reaction that is followed by spontaneous decarboxylation and lactamization reactions that provide active etoposide. The structure of Etoposide-SCS-873 is shown below.

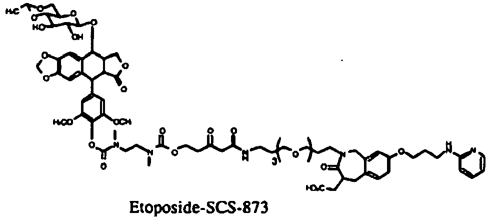

### EXAMPLE 7: Preparation of an Integrin Targeting Compound for Liposome Targeting (not part of the invention)

DoxilTM containing liposomes are associated with a targeting compound of the invention as described. The targeting component is attached to maleimide-terminated PEG2000-DSPE (MAL-PEG2000-DSPE). MAL-PEG2000-DSPE is commercially available through Shearwater Polymers, Inc. Attachment of thiol terminated targeting molecules to the maleimide moiety is spontaneous. The PEG-lipid derivative is then transferred into the DoxilTM liposome with negligible drug leakage in a simple incubation step.

Targeted binding of liposomes to cells is studied by FACS analysis by co-incorporation of biotin labeled MAL-PEG2000-DSPE and staining with FITC-labeled streptavidin. Various control cell lines are also studied to assess non-specific binding. Cytotoxicity assays are performed as described (Moase et al., 2001) with a variety of different loadings of the targeting molecules into the liposome using the three KS cell lines. To study the efficacy of this approach in an SLK animal model, animals are treated 1 day after cell implantation and after tumors have established (200 mm³). A single dosing regimen is studied initially to assess the relative efficacy of targeted Doxil^{™} vs. untargeted Doxil^{™}. Drug dosing ranges from 0.5 mg/kg to 5 mg/kg i.v.. Other control groups are be treated with empty, but targeted liposomes to assess the effect of the multivalent liposome itself on the disease. A buffer control group is also included. In multiple treatment studies, drug is injected at 14-day intervals. Systemic toxicity is assessed as described above.

It should be noted that Doxil^{™} is also approved for the treatment of refractory ovarian cancers. A recent study of 25 permanent human cell lines established from advanced ovarian cancer demonstrated that all lines were positive for integrin expression (Bruning et al., Hum. Gene Ther. 12, 391-399, 2001). This result suggests that the targeting of both tumor and its supporting vasculature also has utility in the treatment of ovarian cancer. Recent studies have indicated that αᵥβ₃ is highly expressed on malignant human cervical tumor tissues (Chattopadhyay and Chatterjee, J. Exp. Clin. Cancer Res. 20, 269-275, 2001), suggesting that the Doxil^{™} based strategy disclosed herein have utility in the treatment of cervical cancer. Other polymerized liposome assemblies such as those described in Bruehl et al. (Biochemistry, 40:5964-5971, 2001) can also be used.

## Claims

1. An integrin targeting compound, comprising at least one integrin targeting component covalently linked to a linear or branched linker which is covalently linked to at least one functional component, wherein said integrin targeting component is an RGD peptidomimetic, and wherein the at least one functional component is an aldolase antibody and the at least one integrin targeting component is covalently linked via the linker to the combining site of the antibody.

2. The targeting compound of claim 1 wherein the integrin targeting component targets α₁β₁, α₂β₁, α₃β₁, α₄β₁, α₅β₁, α₆β₁, α₇β₁, α₈β₁, α₉β₁, α₆β₄, α₄β₇, α_{D}β₂, α_{L}β₂, α_{M}β₂, αᵥβ₁, αᵥβ₃, αᵥβ₅, αᵥβ₆, αᵥβ₈, αₓβ₂, α_{IIb}β₃, or α_{IELb}β₇.

3. The targeting compound of claim 1 wherein said antibody is full length.

4. The targeting compound of claim 1 wherein said antibody is a fragment of a full length antibody.

5. The targeting compound of claim 4 wherein said fragment of a full length antibody is Fab, Fab' F(ab')₂, Fv or sFv.

6. The targeting compound of claim 1 wherein said antibody is a human antibody, humanized antibody or chimeric human antibody.

7. The targeting compound of claim 1 wherein the integrin targeting component is linked to two or more functional components, the first functional component being an antibody.

8. The targeting compound of claim 7 wherein said at least one of said two or more functional components is a therapeutic agent.

9. The targeting compound of claim 8 wherein the therapeutic agent is selected from the group consisting of paclitaxel, doxorubicin, 2-pyyrolinodoxorubicin, and etoposide.

10. The targeting compound of claim 1 wherein the integrin targeting component comprises two or more targeting components.

11. The targeting compound of claim 1 wherein said linker comprises a linear stretch of between 5-100 atoms selected from the group consisting of C, H, N, O, P, S, Si, F, Cl, Br, and I, or a salt thereof.

12. The targeting compound of claim 11 wherein said linker comprises one or more groups selected from alkyl, alkenyl, alkynyl, oxoalkyl, oxoalkenyl, oxoalkynyl, aminoalkyl, aminoalkenyl, aminoalkynyl, sulfoalkyl, sulfoalkenyl, sulfoalkynyl, phosphoalkyl, phosphoalkenyl, and phosphoalkynyl.

13. The targeting compound of claim 1 wherein said linker comprises a repeating ether unit of between 2-100 units.

14. The targeting compound of claim 1 wherein said linker comprises a heterocarbyl structure of the formula 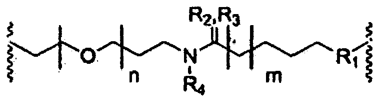 wherein
R₂ to R₄ is C, H, N, O, P, S, Si, halogen (F, Cl, Br, I) or a salt thereof;
n is 1-100; and
m is 1-100.

15. The targeting compound of claim 1, wherein the linker has the formula
X-Y-Z
wherein
X is a linear or branched connecting chain of atoms comprising any of C, H, N, O, P, S, Si, F, Cl, Br, and I, or a salt thereof,
Y if present is a single or fused 5 or 6 membered homo- or heterocarbocylic saturated or unsaturated ring; and
Z is a ketone, diketone, beta lactam, active ester, haloketone, lactone, anhydride, epoxide, aldehyde, maleimide disulfide, or aryl halide; and
wherein Z is a reactive group for covalently linking one of the components to reactive amino acid or other susceptible moiety in the other of the components, said targeting component or functional component linked to X or Y if present or both X and Y if Y is present.

16. A targeting compound as claimed in claim 15, wherein
X is a linear or branched connecting chain of atoms comprising any of C, H, N, O, P, S, Si, F, Cl, Br, and I, or a salt thereof, and comprising a repeating unit of between 2 and 100 units, and Y if present, is a single or fused 5 or 6 membered homo-or heterocarbocylic saturated or unsaturated ring and is located within 1-20 atoms of Z.

17. The targeting compound of claim 15 or 16, wherein said components are linked in such a way to retain the ability to bind the target and exhibit functional activity.

18. The targeting compound of claim 15 or 16, wherein X comprises a linear stretch of between 5-200 atoms.

19. The targeting compound of claim 15 or 16, wherein X is a heterocarbyl structure of the formula 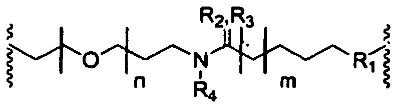 wherein
R₂ to R₄ is C, H, N, O, P, S, Si, halogen (F, Cl, Br, I) or a salt thereof;
n is 1-100; and
m is 1-100.

20. The targeting compound of claim 15 or 16, wherein Y is a six membered ring of the formula 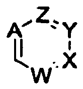 wherein
A, Z, Y, X or W are independently C or N.

21. The targeting compound of claim 15 or 16, wherein Y is a five membered ring of the formula 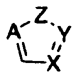 wherein
A, Z, Y or X are independently C, O, N or S.

22. The targeting compound of claim 15 or 16, wherein said linker is branched by addition of one or more connecting chains, said linker comprises more than one recognition group, said linker comprises more than one reactive group, or combinations thereof.

23. The targeting compound of claim 15 or 16, wherein said linker has the structure below wherein n is from 1-100.

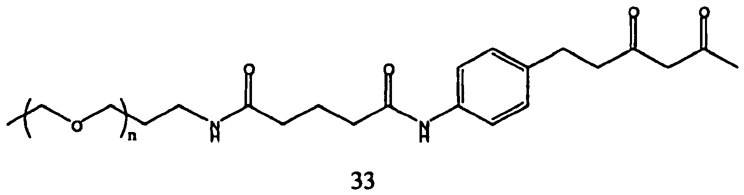

24. The targeting compound of claim 15 or 16, wherein said linker comprises more than one connecting chain, more than one recognition group or more than one reactive group, or combinations thereof.

25. The targeting compound of claim 1 wherein said integrin targeting component is an RGD peptidomimetic shown as compounds 1, 2 or 3, below.

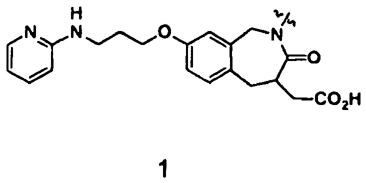

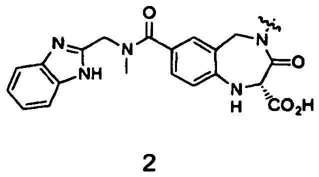

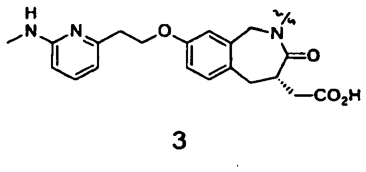

26. The targeting compound of claim 1 wherein said covalent linkage between said targeting component and said linker or between said linker and said functional component or both is nonreversible.

27. The targeting compound of claim 1 wherein said covalent linkage between said targeting component and said linker or between said linker and said functional component or both is reversible.

28. The targeting compound of claim 1 wherein said covalent linkage between said targeting component and said linker or between said linker and said functional component or both is labile.

29. The targeting compound of claim 21 wherein said labile linkage is a pH sensitive linkage, is a substrate for an enzyme, or is susceptible to degradation by radiation.

30. A method of producing an integrin targeting compound, comprising covalently linking at least one integrin targeting component via a linear or branched linker to at least one functional component, wherein said integrin targeting component is an RGD peptidomimetic, and wherein the at least one functional component is an aldolase antibody and the at least one integrin targeting component is covalently linked via the linker to the combining site of the antibody.

31. The method of claim 30 wherein said at least one targeting component is linked to said at least one functional component in such a way as to retain the binding function of the targeting component and the biological activity of the functional component.

32. The method of claim 30 wherein said linking is achieved by preparing an integrin targeting component-linker compound comprising said at least one integrin targeting component and a linker, said linker comprising a reactive group for reaction with the functional component, and linking said reactive group of said linker covalently to the functional component.

33. The method of claim 30 wherein said linking is achieved by preparing a functional component-linker compound comprising a functional component and a linker, said linker comprising a reactive group for reaction with said at least one integrin targeting component, and linking the reactive group of said linker covalently to said at least one integrin targeting component.

34. The method of claim 30 wherein said linking is achieved by:
(a) preparing an integrin targeting component-linker compound comprising an integrin targeting component and a linker comprising a reactive group; and
(b) preparing a functional component-linker comprising a functional compound and a linker comprising a chemical moiety susceptible to reaction with the reactive group of step (a); or
(c) preparing a functional component-linker compound comprising a functional component and a linker comprising a reactive group; and
(d) preparing an integrin targeting component-linker compound comprising an integrin targeting component and a linker comprising a chemical moiety susceptible to reaction with said reactive group of step (c); and
(e) linking the linkers of steps (a) and (b) or steps (c) and (d) covalently together through said reactive and susceptible groups to form the integrin targeting compound.

35. A targeting compound of claim 1, for use in the treatment of a disease or condition that is associated with the integrin being targeted.

36. The targeting compound of claim 35 wherein said disease or condition involves a defect in angiogenesis, bone metabolism, inflammation or cell growth.

37. The targeting compound of claim 35 wherein said disease or condition is cancer.

38. A pharmaceutical formulation comprising the targeting compound of claim 1 and a pharmaceutically acceptable carrier.

## Patentansprüche

1. Auf Integrin zielende Verbindung, umfassend wenigstens eine mit einem geradlinigen oder verzweigten Linker, der kovalent mit wenigstens einer funktionellen Komponente verknüpft ist, kovalent verknüpfte auf Integrin zielende Komponente, wobei es sich bei der auf Integrin zielenden Komponente um ein RGD-Peptidomimetikum und bei der wenigstens einen funktionellen Komponente um einen Aldolase-Antikörper handelt und die wenigstens eine auf Integrin zielende Komponente über den Linker kovalent mit der Bindungsstelle des Antikörpers verknüpft ist.

2. Zielende Verbindung nach Anspruch 1, wobei die auf Integrin zielende Komponente auf α₁β₁, α₂β₁, α₃β₁, α₄β₁, α₅β₁, α₆β₁, α₇β₁, α₈β₁, α₉β₁, α₆β₄, α₄β₇, α_{D}β₂, α_{L}β₂, α_{M}β₂, αᵥβ₁, αᵥβ₃, αᵥβ₅, αᵥβ₆, αᵥβ₈, αₓβ₂, α_{IIb}β₃ oder α_{IELb}β₇ zielt.

3. Zielende Verbindung nach Anspruch 1, wobei der Antikörper volle Länge besitzt.

4. Zielende Verbindung nach Anspruch 1, wobei es sich bei dem Antikörper um ein Fragment eines Volllängen-Antikörpers handelt.

5. Zielende Verbindung nach Anspruch 4, wobei es sich bei dem Fragment eines Volllängen-Antikörpers um Fab, Fab`, F(ab')₂, Fv oder sFv handelt.

6. Zielende Verbindung nach Anspruch 1, wobei es sich bei dem Antikörper um einen menschlichen Antikörper, humanisierten Antikörper oder chimären menschlichen Antikörper handelt.

7. Zielende Verbindung nach Anspruch 1, wobei die auf Integrin zielende Komponente mit zwei oder mehr funktionellen Komponenten verknüpft ist, wobei es sich bei der ersten funktionellen Komponente um einen Antikörper handelt.

8. Zielende Verbindung nach Anspruch 7, wobei es sich bei der wenigstens einen der zwei oder mehr funktionellen Komponenten um ein Therapeutikum handelt.

9. Zielende Verbindung nach Anspruch 8, wobei das Therapeutikum ausgewählt ist aus der Gruppe Paclitaxel, Doxorubicin, 2-Pyrrolinodoxorubicin und Etoposid.

10. Zielende Verbindung nach Anspruch 1, wobei die auf Integrin zielende Komponente zwei oder mehr zielende Komponenten umfasst.

11. Zielende Verbindung nach Anspruch 1, wobei der Linker einen geradlinigen Abschnitt mit 5-100 Atomen, ausgewählt aus der Gruppe C, H, N, O, P, S, Si, F, Cl, Br und I oder einem Salz davon, umfasst.

12. Zielende Verbindung nach Anspruch 11, wobei der Linker eine oder mehrere Gruppen, ausgewählt aus Alkyl, Alkenyl, Alkinyl, Oxoalkyl, Oxoalkenyl, Oxoalkinyl, Aminoalkyl, Aminoalkenyl, Aminoalkenyl, Sulfoalkyl, Sulfoalkenyl, Sulfoalkinyl, Phosphoalkyl, Phosphoalkenyl und Phosphoalkinyl, umfasst.

13. Zielende Verbindung nach Anspruch 1, wobei der Linker eine sich wiederholende Ethereinheit mit 2-100 Einheiten umfasst.

14. Zielende Verbindung nach Anspruch 1, wobei der Linker eine Heterokohlenwasserstoffstruktur der Formel 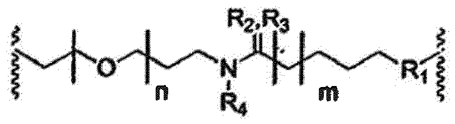 umfasst, wobei
R₂ bis R₄ für C, H, N, O, P, S, Si, Halogen (F, Cl, Br, I) oder ein Salz davon steht;
n gleich 1-100 ist; und
m gleich 1-100 ist.

15. Zielende Verbindung nach Anspruch 1, wobei der Linker die Formel
X-Y-Z
aufweist, wobei
X für eine geradlinige oder verzweigte Verbindungskette von Aromen, beliebig umfassend C, H, N, O, P, S, Si, F, Cl, Br und I oder ein Salz davon, steht,
Y, falls vorhanden, für einen einzelnen oder kondensierten 5- oder 6-gliedrigen homo- oder heterocarbocyclischen gesättigten oder ungesättigten Ring steht; und
Z für ein Keton, Diketon, Beta-Lactam, einen aktiven Ester, ein Halogenketon, Lacton, Anhydrid, Epoxid, einen Aldehyd, Maleimiddisulfid oder ein Arylhalogenid steht; und
wobei es sich bei Z um eine reaktive Gruppe zur kovalenten Verknüpfung einer der Komponenten mit einer reaktiven Aminosäure oder anderen reaktionsfähigen Gruppierung in der anderen Komponente handelt, wobei die zielende Komponente oder funktionelle Komponente mit X oder Y, falls vorhanden, oder sowohl X als auch Y, wenn Y vorhanden ist, verknüpft ist.

16. Zielende Verbindung gemäß Anspruch 15, wobei
X für eine geradlinige oder verzweigte Verbindungskette von Atomen, beliebig umfassend C, H, N, O, P, S, Si, F, Cl, Br und I oder ein Salz davon und umfassend eine sich wiederholende Einheit mit zwischen 2 und 100 Einheiten, steht und Y, falls vorhanden, für einen einzelnen oder kondensierten 5- oder 6-gliedrigen homo- oder heterocarbocyclischen gesättigten oder ungesättigten Ring steht und innerhalb von 1-20 Atomen von Z liegt.

17. Zielende Verbindung nach Anspruch 15 oder 16, wobei die Komponenten so verknüpft sind, dass sie die Fähigkeit zur Bindung des Ziels behalten und funktionelle Aktivität zeigen.

18. Zielende Verbindung nach Anspruch 15 oder 16, wobei X einen geradlinigen Abschnitt mit 5-200 Atomen umfasst.

19. Zielende Verbindung nach Anspruch 15 oder 16, wobei X für eine Heterokohlenwasserstoffstruktur der Formel 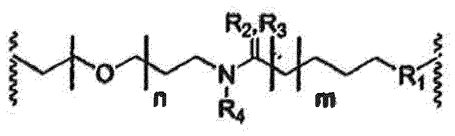 steht, wobei
R₂ bis R₄ für C, H, N, O, P, S, Si, Halogen (F_{,} Cl, Br, I) oder ein Salz davon steht;
n gleich 1-100 ist; und
m gleich 1-100 ist.

20. Zielende Verbindung nach Anspruch 15 oder 16, wobei Y für einen sechsgliedrigen Ring der Formel 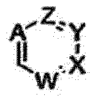 steht, wobei
A, Z, Y, X oder W unabhängig für C oder N stehen.

21. Zielende Verbindung nach Anspruch 15 oder 16, wobei Y für einen fünfgliedrigen Ring der Formel 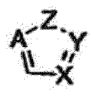 steht, wobei A, Z, Y oder X unabhängig für C, 0, N oder S stehen.

22. Zielende Verbindung nach Anspruch 15 oder 16, wobei der Linker durch Addition einer oder mehrerer Verbindungsketten verzweigt wird, der Linker mehr als eine Erkennungsgruppe umfasst, der Linker mehr als eine reaktive Gruppe umfasst oder Kombinationen davon.

23. Zielende Verbindung nach Anspruch 15 oder 16, wobei der Linker die nachfolgende Struktur aufweist, wobei n gleich 1-100 ist.

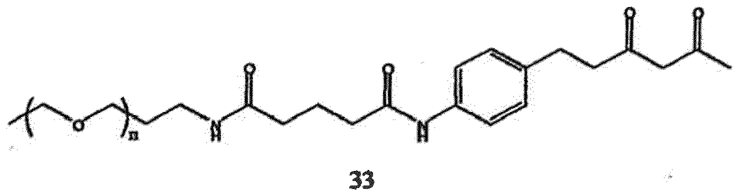

24. Zielende Verbindung nach Anspruch 15 oder 16, wobei der Linker mehr als eine Verbindungskette, mehr als eine Erkennungsgruppe oder mehr als eine reaktive Gruppe oder Kombinationen davon umfasst.

25. zielende Verbindung nach Anspruch 1, wobei es sich bei der auf Integrin zielenden Komponente um ein nachfolgend als Verbindung 1, 2 bzw. 3 dargestelltes RGD-Peptidomimetikum handelt.

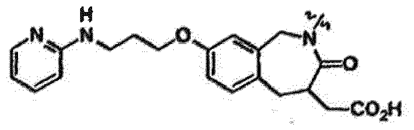

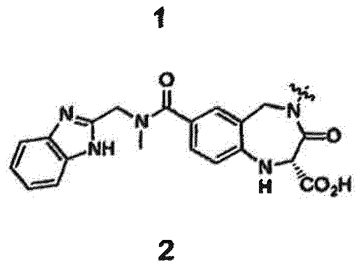

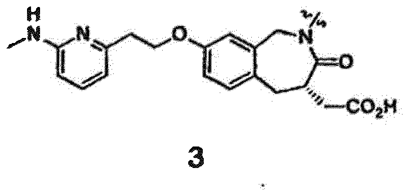

26. Zielende Verbindung nach Anspruch 1, wobei die kovalente Verknüpfung zwischen der zielenden Komponente und dem Linker oder/und zwischen dem Linker und der funktionellen Komponente nicht reversibel ist.

27. Zielende Verbindung nach Anspruch 1, wobei die kovalente Verknüpfung zwischen der zielenden Komponente und dem Linker oder/und zwischen dem Linker und der funktionellen Komponente reversibel ist.

28. Zielende Verbindung nach Anspruch 1, wobei die kovalente Verknüpfung zwischen der zielenden Komponente und dem Linker oder/und zwischen dem Linker und der funktionellen Komponente labil ist.

29. Zielende Verbindung nach Anspruch 21, wobei es sich bei der labilen Verknüpfung um eine pHempfindliche Verknüpfung oder um ein Substrat für ein Enzym handelt oder die labile Verknüpfung anfällig für einen Abbau durch Strahlung ist.

30. Verfahren zur Herstellung einer auf Integrin zielenden Verbindung, bei dem man wenigstens eine auf Integrin zielende Komponente über einen geradlinigen oder verzweigten Linker kovalent mit wenigstens einer funktionellen Komponente verknüpft, wobei es sich bei der auf Integrin zielenden Komponente um ein RGD-Peptidomimetikum und bei der wenigstens einen funktionellen Komponente um einen Aldolase-Antikörper handelt und die wenigstens eine auf Integrin zielende Komponente über den Linker kovalent mit der Bindungsstelle des Antikörpers verknüpft ist.

31. Verfahren nach Anspruch 30, wobei die wenigstens eine zielende Komponente mit der wenigstens einen funktionellen Komponente so verknüpft ist, dass die Bindungsfunktion der zielenden Komponente und die biologische Aktivität der funktionellen Komponente erhalten bleiben.

32. Verfahren nach Anspruch 30, wobei die Verknüpfung dadurch erreicht wird, dass man eine Auf-Integrinzielende-Komponente-Linker-Verbindung, die wenigstens eine auf Integrin zielende Komponente und einen Linker umfasst, wobei der Linker eine reaktive Gruppe zur Reaktion mit der funktionellen Komponente umfasst, herstellt und die reaktive Gruppe des Linkers kovalent mit der funktionellen Komponente verknüpft.

33. Verfahren nach Anspruch 30, wobei die Verknüpfung dadurch erreicht wird, dass man eine Funktionelle-Komponente-Linker-Verbindung, die eine funktionelle Komponente und einen Linker umfasst, wobei der Linker eine reaktive Gruppe zur Reaktion mit der wenigstens einen auf Integrin zielenden Komponente umfasst, herstellt und die reaktive Gruppe des Linkers kovalent mit der wenigstens einen auf Integrin zielenden Komponente verknüpft.

34. Verfahren nach Anspruch 30, wobei die Verknüpfung dadurch erreicht wird, dass man:
a. eine Auf-Integrin-zielende-Komponente-Linker-Verbindung, die eine auf Integrin zielende Komponente und einen eine reaktive Gruppe umfassenden Linker umfasst, herstellt; und
b. einen Funktionelle-Komponente-Linker, der eine funktionelle Verbindung und einen eine der Reaktion mit der reaktiven Gruppe aus Schritt (a) zugängliche chemische Gruppierung umfassenden Linker umfasst, herstellt; oder
c. eine Funktionelle-Komponente-Linker-Verbindung, die eine funktionelle Komponente und einen eine reaktive Gruppe umfassenden Linker umfasst, herstellt; und
d. eine Auf-Integrin-zielende-Komponente-Linker-Verbindung, die eine auf Integrin zielende Komponente und einen eine der Reaktion mit der reaktiven Gruppe aus Schritt (c) zugängliche chemische Gruppierung umfassenden Linker umfasst, herstellt; und
e. die Linker aus Schritten (a) und (b) oder aus Schritten (c) und (d) über die reaktiven bzw. zugänglichen Gruppen unter Bildung der auf Integrin zielenden Verbindung kovalent miteinander verknüpft.

35. Zielende Verbindung nach Anspruch 1, zur Verwendung bei der Behandlung einer Krankheit oder eines Leidens, die bzw. das in Zusammenhang mit als Ziel dienenden Integrin steht.

36. Zielende Verbindung nach Anspruch 35, wobei die Krankheit bzw. das Leider einen Defekt bei der Angiogenese, dem Knochenstoffwerhsel, Entzündung oder Zellwachstum beinhaltet.

37. Zielende Verbindung nach Anspruch 35, wobei es sich bei der Krankheit bzw. dem Leiden um Krebs handelt.

38. Pharmazeutische Formulierung, umfassend die zielende Verbindung nach Anspruch 1 sowie einen pharmazeutisch unbedenkliches Trägerstoff.

## Revendications

1. Composé de ciblage d'intégrine, comprenant au moins un composant de ciblage d'intégrine lié de façon covalente à un lieur linéaire ou ramifié qui est lié de façon covalente à au moins un composant fonctionnel, où ledit composant de ciblage d'intégrine est un peptidomimétique RGD, et où l'au moins un composant fonctionnel est un anticorps anti-aldolase et l'au moins un composant de ciblage d'intégrine est lié de façon covalente par l'intermédiaire du lieur au site de combinaison de l'anticorps.

2. Composé de ciblage de la revendication 1 dans lequel le composant de ciblage d'intégrine cible α₁β₁, α₂β₁, α₃β₁, α₄β₁, α₅β₁, α₆β₁, α₇β₁, α₈β₁, α₉β₁, α₆β₄, α₄β₇, α_{D}β₂, α_{L}β₂, α_{M}β₂, αᵥβ₁, αᵥβ₃, αᵥβ₅, αᵥβ₆, αᵥβ₈, αₓβ₂, α_{IIb}β₃, ou α_{IELb}β₇.

3. Composé de ciblage de la revendication 1 dans lequel ledit anticorps est de longueur totale.

4. Composé de ciblage de la revendication 1 dans lequel ledit anticorps est un fragment d'un anticorps de longueur totale.

5. Composé de ciblage de la revendication 4 où ledit fragment d'un anticorps de longueur totale est Fab, Fab', F(ab')₂, Fv ou sFv.

6. Composé de ciblage de la revendication 1 dans lequel ledit anticorps est un anticorps humain, un anticorps humanisé ou un anticorps humain chimérique.

7. Composé de ciblage de la revendication 1 dans lequel le composant de ciblage d'intégrine est lié à deux composants fonctionnels ou plus, le premier composant fonctionnel étant un anticorps.

8. Composé de ciblage de la revendication 7 où ledit au moins un desdits deux composants fonctionnels ou plus est un agent thérapeutique.

9. Composé de ciblage de la revendication 8 où l'agent thérapeutique est choisi dans le groupe constitué du paclitaxel, de la doxorubicine, de la 2-pyrrolinodoxorubicine, et de l'étoposide.

10. Composé de ciblage de la revendication 1 dans lequel le composant de ciblage d'intégrine comprend deux composants de ciblage ou plus.

11. Composé de ciblage de la revendication 1 dans lequel ledit lieur comprend un segment linéaire compris entre 5 et 100 atomes choisis dans le groupe constitué de C, H, N, O, P, S, Si, F, Cl, Br, et I, ou un sel de celui-ci.

12. Composé de ciblage de la revendication 11 dans lequel ledit lieur comprend un ou plusieurs groupes choisis parmi alkyle, alcényle, alcynyle, oxoalkyle, oxoalcényle, oxoalcynyle, aminoalkyle, aminoalcényle, aminoalcynyle, sulfoalkyle, sulfoalcényle, sulfoalcynyle, phosphoalkyle, phosphoalcényle, et phosphoalcynyle.

13. Composé de ciblage de la revendication 1 dans lequel ledit lieur comprend un motif éther de répétition compris entre 2 et 100 motifs.

14. Composé de ciblage de la revendication 1 dans lequel ledit lieur comprend une structure hétérocarbyle de formule 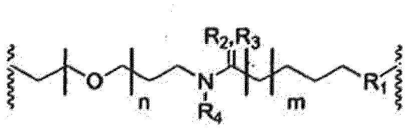 dans laquelle
R₂ à R₄ est C, H, N, 0, P, S, Si, un halogène (F, Cl, Br, I) ou un sel de celui-ci ;
n est de 1 à 100 ; et
m est de 1 à 100.

15. Composé de ciblage de la revendication 1, où le lieur a la formule
X-Y-Z
dans laquelle
X est une chaîne de connexion linéaire ou ramifiée d'atomes comprenant l'un quelconque de C, H, N, O, P, S, Si, F, Cl, Br, et I, ou un sel de celui-ci,
Y s'il est présent est un cycle saturé ou insaturé homo- ou hétérocarbocyclique de 5 ou 6 chaînons individuel ou condensé ; et
Z est une cétone, une dicétone, un bêta-lactame, un ester actif, une halogénocétone, une lactone, un anhydride, un époxyde, un aldéhyde, un disulfure de maléimide, ou un halogénure d'aryle ; et
où Z est un groupe réactif pour lier de façon covalente l'un des composants à un acide aminé réactif ou un autre fragment susceptible dans l'autre des composants, ledit composant de ciblage ou composant fonctionnel étant lié à X ou Y s'il est présent ou à la fois à X et Y si Y est présent.

16. Composé de ciblage selon la revendication 15, dans lequel
X est une chaîne de connexion linéaire ou ramifiée d'atomes comprenant l'un quelconque de C, H, N, O, P, S, Si, F, Cl, Br, et I, ou un sel de celui-ci, et comprenant un motif de répétition compris entre 2 et 100 unités, et Y s'il est présent, est un cycle saturé ou insaturé homo- ou hétérocarbocyclique de 5 ou 6 chaînons individuel ou condensé et est situé à 1 à 20 atomes de Z.

17. Composé de ciblage de la revendication 15 ou 16, où lesdits composants sont liés de manière à conserver la capacité à se lier à la cible et présenter une activité fonctionnelle.

18. Composé de ciblage de la revendication 15 ou 16, où X comprend un segment linéaire compris entre 5 et 200 atomes.

19. Composé de ciblage de la revendication 15 ou 16, où X est une structure hétérocarbyle de formule 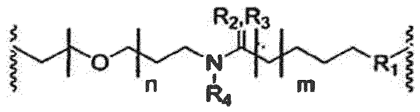 dans laquelle
R₂ à R₄ est C, H, N, 0, P, S, Si, un halogène (F, Cl, Br, I) ou un sel de celui-ci ;
n est de 1 à 100 ; et
m est de 1 à 100.

20. Composé de ciblage de la revendication 15 ou 16, où Y est un cycle de six chaînons de formule 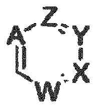 dans laquelle
A, Z, Y, X ou W sont indépendamment C ou N.

21. Composé de ciblage de la revendication 15 ou 16, où Y est un cycle de cinq chaînons de formule 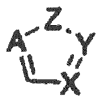 dans laquelle
A, Z, Y ou X sont indépendamment C, O, N ou S.

22. Composé de ciblage de la revendication 15 ou 16, dans lequel ledit lieur est ramifié par ajout d'une ou plusieurs chaînes de connexion, ledit lieur comprend plus d'un groupe de reconnaissance, ledit lieur comprend plus d'un groupe réactif, ou des combinaisons de ceux-ci.

23. Composé de ciblage de la revendication 15 ou 16, où ledit lieur a la structure ci-dessous dans laquelle n est de 1 à 100.

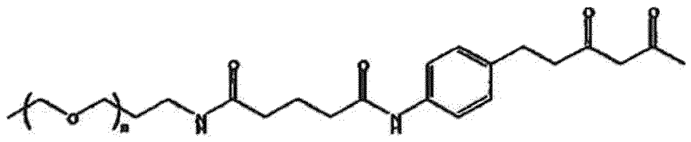

24. Composé de ciblage de la revendication 15 ou 16, où ledit lieur comprend plus d'une chaîne de connexion, plus d'un groupe de reconnaissance ou plus d'un groupe réactif, ou des combinaisons de ceux-ci.

25. Composé de ciblage de la revendication 1 dans lequel ledit composant de ciblage d'intégrine est un peptidomimétique RGD représenté par les composés 1, 2 ou 3, ci-dessous.

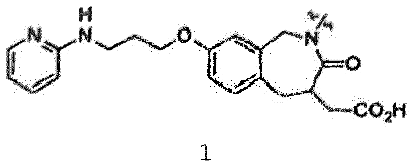

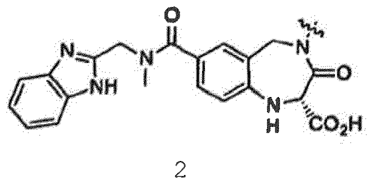

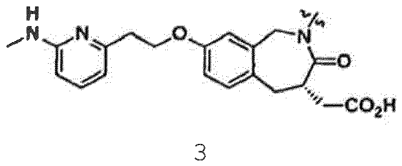

26. Composé de ciblage de la revendication 1 dans lequel ladite liaison covalente entre ledit composant de ciblage et ledit lieur ou entre ledit lieur et ledit composant fonctionnel ou les deux est non réversible.

27. Composé de ciblage de la revendication 1 dans lequel ladite liaison covalente entre ledit composant de ciblage et ledit lieur ou entre ledit lieur et ledit composant fonctionnel ou les deux est réversible.

28. Composé de ciblage de la revendication 1 dans lequel ladite liaison covalente entre ledit composant de ciblage et ledit lieur ou entre ledit lieur et ledit composant fonctionnel ou les deux est labile.

29. Composé de ciblage de la revendication 21 dans lequel ladite liaison labile est une liaison sensible au pH, est un substrat pour une enzyme, ou est sensible à la dégradation par rayonnement.

30. Procédé de production d'un composé de ciblage d'intégrine, comprenant la liaison de façon covalente d'au moins un composant de ciblage d'intégrine via un lieur linéaire ou ramifié à au moins un composant fonctionnel, où ledit composant de ciblage d'intégrine est un peptidomimétique RGD, et où l'au moins un composant fonctionnel est un anticorps anti-aldolase et l'au moins un composant de ciblage d'intégrine est lié de façon covalente via le lieur au site de combinaison de l'anticorps.

31. Procédé de la revendication 30 dans lequel ledit au moins un composant de ciblage est lié audit au moins un composant fonctionnel de manière à conserver la fonction de liaison du composant de ciblage et l'activité biologique du composant fonctionnel.

32. Procédé de la revendication 30 dans lequel ladite liaison est obtenue par préparation d'un composant de ciblage d'intégrine-composé lieur comprenant ledit au moins un composant de ciblage d'intégrine et un lieur, ledit lieur comprenant un groupe réactif pour réaction avec le composant fonctionnel, et liaison dudit groupe réactif dudit lieur de façon covalente au composant fonctionnel.

33. Procédé de la revendication 30 dans lequel ladite liaison est obtenue par préparation d'un composant fonctionnel-composé lieur comprenant un composant fonctionnel et un lieur, ledit lieur comprenant un groupe réactif pour réaction avec ledit au moins un composant de ciblage d'intégrine, et liaison du groupe réactif dudit lieur de façon covalente audit au moins un composant de ciblage d'intégrine.

34. Procédé de la revendication 30 dans lequel ladite liaison est obtenue par :
(a) préparation d'un composant de ciblage d'intégrine-composé lieur comprenant un composant de ciblage d'intégrine et un lieur comprenant un groupe réactif ; et
(b) préparation d'un composant fonctionnel-lieur comprenant un composé fonctionnel et un lieur comprenant un fragment chimique susceptible de réaction avec le groupe réactif de l'étape (a) ; ou
(c) préparation d'un composant fonctionnel-composé lieur comprenant un composant fonctionnel et un lieur comprenant un groupe réactif ; et
(d) préparation d'un composant de ciblage d'intégrine-composé lieur comprenant un composant de ciblage d'intégrine et un lieur comprenant un fragment chimique susceptible de réaction avec ledit groupe réactif de l'étape (c) ; et
(e) liaison des lieurs des étapes (a) et (b) ou étapes (c) et (d) de façon covalente conjointement par l'intermédiaire desdits groupes réactifs et susceptibles pour former le composé de ciblage d'intégrine.

35. composé de ciblage de la revendication 1, pour utilisation dans le traitement d'une maladie ou affection qui est associée à l'intégrine étant ciblée.

36. Composé de ciblage de la revendication 35 où ladite maladie ou affection met en oeuvre un défaut dans l'angiogenèse, le métabolisme osseux, l'inflammation ou la croissance cellulaire.

37. Composé de ciblage de la revendication 35 où ladite maladie ou affection est le cancer.

38. Formulation pharmaceutique comprenant le composé de ciblage de la revendication 1 et un véhicule pharmaceutiquement acceptable.
